# EUROPEAN PATENT APPLICATION

(11) **EP 2 387 999 A1**
(43) Date of publication of application: **23.11.2011**
(21) Application number: 10005373.5
(22) Date of filing: 21.05.2010
(51) Int. Cl.: A61K 31/4172, A61K 47/18, A61K 48/00

(54) **Histidine-containing solution for transfection and/or injection of nucleic acids and uses thereof**

(71) Applicant: CureVac GmbH, 72076 Tübingen (DE)
(72) Inventor: Gorlovoy, Philipp, Dr., 72076 Tübingen (DE); Baumhof, Patrick, Dr., 72144 Dusslingen (DE); Schlake, Thomas, Dr., 79194 Gundelfingen (DE)
(74) Representative: Graf von Stosch, Andreas

(57) **Abstract**

The present invention is directed to a solution for transfection and/or injection containing at least one nucleic acid (sequence) and (free) histidine in a concentration of about 1 to about 25 µM. The present invention is also directed to the use of such histidine-containing solutions for transfection and/or injection of nucleic acid(s) (sequences), in particular for improvement of the transfection efficiency and/or endosomal release of nucleic acids. It is also directed to pharmaceutical compositions comprising such an inventive solution and its use as a medicament and/or for the prophylaxis, the treatment and/or the amelioration of various diseases as mentioned herein, e.g. by vaccination. The present invention is also directed to a method for transfection of a cell or an organism utilizing these inventive solutions and pharmaceutical compositions and to kits, comprising the inventive solution or pharmaceutical compositions thereof.

<110>  CureVac GmbH

<120>  Lyophilization of nucleic acids in lactate-containing solutions

<130>  CU01P089EP

<160>  2

<170>  PatentIn version 3.5

<210>  1
<211>  1857
<212>  RNA
<213>  Artificial Sequence

<220>
<223>  mRNA sequence termed pCV19-Pp luc(GC)-muag-A70-C30 coding for
       Photinus pyralis luciferase

<400>  1
gggagaaagc uugaggaugg aggacgccaa gaacaucaag aagggcccgg cgcccuucua      60

cccgcuggag gacgggaccg ccggcgagca gcuccacaag gccaugaagc gguacgcccu     120

ggugccgggc acgaucgccu ucaccgacgc ccacaucgag gucgacauca ccuacgcgga     180

guacuucgag augagcgugc gccuggccga ggccaugaag cgguacggcc ugaacaccaa     240

ccaccggauc guggugugcu cggagaacag ccugcaguuc uucaugccgg ugcugggcgc     300

ccucuucauc ggcguggccg ucgccccggc gaacgacauc uacaacgagc gggagcugcu     360

gaacagcaug gggaucagcc agccgaccgu gguguucgug agcaagaagg gccugcagaa     420

gauccugaac gugcagaaga agcugcccau cauccagaag aucaucauca uggacagcaa     480

gaccgacuac cagggcuucc agucgaugua cacguucgug accagccacc ucccgccggg     540

cuucaacgag uacgacuucg ucccggagag cuucgaccgg gacaagacca ucgcccugau     600

caugaacagc agcggcagca ccggccugcc gaagggggug gcccugccgc accggaccgc     660

cugcgugcgc uucucgcacg cccgggaccc caucuucggc aaccagauca ucccggacac     720

cgccauccug agcguggugc cguuccacca cggcuucggc auguucacga cccugggcua     780

ccucaucugc ggcuuccggg ugguccugau guaccgguuc gaggaggagc uguuccugcg     840

gagccugcag gacuacaaga uccagagcgc gcugcucgug ccgacccugu ucagcuucuu     900

cgccaagagc acccugaucg acaaguacga ccugucgaac cugcacgaga ucgccagcgg     960

gggcgccccg cugagcaagg aggugggcga ggccguggcc aagcgguucc accucccggg    1020

cauccgccag ggcuacggcc ugaccgagac cacgagcgcg auccugauca cccccgaggg    1080

ggacgacaag ccgggcgccg ugggcaaggu ggucccguuc uucgaggcca agguggugga    1140

ccuggacacc ggcaagaccc ugggcgugaa ccagcggggc gagcugugcg ugcgggggcc    1200

gaugaucaug agcggcuacg ugaacaaccc ggaggccacc aacgcccuca ucgacaagga    1260

cggcuggcug cacagcggcg acaucgccua cugggacgag gacgagcacu ucuucaucgu    1320

cgaccggcug aagucgcuga ucaaguacaa gggcuaccag guggcgccgg ccgagcugga    1380

gagcauccug cuccagcacc ccaacaucuu cgacgccggc guggccgggc ugccggacga    1440

cgacgccggc gagcugccgg ccgcgguggu ggugcuggag cacggcaaga ccaugacgga     1500

gaaggagauc gucgacuacg uggccagcca ggugaccacc gccaagaagc ugcggggcgg     1560

cgugguguuc guggacgagg ucccgaaggg ccugaccggg aagcucgacg cccggaagau     1620

ccgcgagauc cugaucaagg ccaagaaggg cggcaagauc gccguguaag acuaguuaua     1680

agacugacua gcccgauggg ccucccaacg ggcccuccuc cccuccuugc accgagauua     1740

auaaaaaaaa aaaaaaaaaa aaaaaaaaaa aaaaaaaaaa aaaaaaaaaa aaaaaaaaaa     1800

aaaaaauauu cccccccccc cccccccccc cccccccccc ucuagacaau uggaauu     1857

<210>  2
<211>  15
<212>  RNA
<213>  Artificial

<220>
<223>  Description of sequence: generic stabilizing sequence of the
       formula (C/U)CCANxCCC(U/A)PyxUC(C/U)CC

<220>
<221>  variation
<222>  (1)..(1)
<223>  /replace="cytosine"
       /replace="uracile"

<220>
<221>  variation
<222>  (5)..(5)
<223>  /replace="cytosine"
       /replace="uracile"
       /replace="guanosine"
       /replace="adenosine"

<220>
<221>  repeat_unit
<222>  (5)..(5)
<223>  x = any number

<220>
<221>  variation
<222>  (9)..(9)
<223>  /replace="uracile"
       /replace="adonosine"

<220>
<221>  repeat_unit
<222>  (10)..(10)
<223>  x = any number

<220>
<221>  variation
<222>  (10)..(10)
<223>  /replace="pyrimidine"

<220>
<221>  variation

<222>  (13)..(13)
<223>  /replace="cytosine"
       /replace="uracile"

<400>  2
nccancccnn ucncc                                                        15

## Description

The present invention is directed to a solution for transfection and/or injection containing at least one nucleic acid (sequence) and (free) histidine in a concentration of about 1 to about 25 µM. The present invention is also directed to the use of such histidine-containing solutions for transfection and/or injection of nucleic acid(s) (sequences), in particular for improvement of the transfection efficiency and/or endosomal release of nucleic acids. It is also directed to pharmaceutical compositions comprising such an inventive solution and its use as a medicament and/or for the prophylaxis, the treatment and/or the amelioration of various diseases as mentioned herein, e.g. by vaccination. The present invention is also directed to a method for transfection of a cell or an organism utilizing these inventive solutions and pharmaceutical compositions and to kits, comprising the inventive solution or pharmaceutical compositions thereof.

In gene therapy and many other therapeutically relevant biochemical and biotechnological applications the use of nucleic acids for therapeutic and diagnostic purposes is of major importance. As an example, rapid progress has occurred in recent years in the field of gene therapy and promising results have been achieved. Nucleic acids are therefore regarded as important tools for gene therapy and prophylactic and therapeutic vaccination against infectious and malignant diseases.

Various diseases today require a treatment which involves administration of peptide-, protein-, and nucleic acid-based drugs, particularly the transfection of nucleic acids into cells or tissues. The full therapeutic potential of peptide-, protein-, and nucleic acid-based drugs is frequently compromised by their limited ability to cross the plasma membrane of mammalian cells, resulting in poor cellular access and inadequate therapeutic efficacy. Today this hurdle represents a major challenge for the biomedical development and commercial success of many biopharmaceuticals (Foerg, C. and H. P. Merkle (2008), J Pharm Sci 97(1): 144-62).

Genetic vaccinations, which consist in the injection of naked plasmid DNA, were demonstrated for the first time in the early 1990s on mice. However, it became clear during clinical phase I/II studies that, in humans, this technology was unable to fulfil the expectations awakened by the studies in mice (Ulmer, J. B. (2001), Curr Opin Drug Discov Devel 4(2): 192-7). Numerous DNA-based genetic vaccinations and methods for introducing DNA into cells (*inter alia* calcium phosphate transfection, polyprene transfection, protoplast fusion, electroporation, microinjection, lipofection, use of DNA viruses as DNA vehicles) have since been developed.

For a successful transfer of nucleic acids or genes into an individual's cells in high efficiency, a number of different hurdles have to be passed. Among them, the transport of nucleic acids is of utmost relevance. It typically occurs via association of the nucleic acid with the cell membrane and the subsequent uptake by endosomes (Mudhakir, D. and H. Hara-shima (2009), Aaps J 11(1): 65-77). In endosomes, the introduced nucleic acids are separated from the cytosol and cannot exhibit any activity. It is thus important for these nucleic acids depart into the cytosol (endosomal escape). If the nucleic acids do not manage to depart into the cytosol, either the endosome fuses with the lysosome leading to a degradation of its content, or the endosome again fuses with the cell membrane leading to a return of its content into the extracellular medium. Endosomal escape thus appears to be one of the most important steps for efficient transfer of nucleic acids (along with an effective transfection).

Until now, there are different approaches addressing the problem of endosomal escape: According to one approach, many polycation gene delivery systems were developed to mimic viral-like mechanisms for endosomal escape. These delivery systems are often coupled with synthetic amphipathic peptides mimicking viral fusogenic peptides, polycations with intrinsic endosomolytic activity by the proton sponge mechanism, polyanions to mimic the anionic amphiphilic peptides and histidine-based gene delivery systems for pH-responsive endosomal escape (Cho, Y. W., J. D. Kim, et al. (2003), J Pharm Pharmacol 55(6): 721-34).

According to a further approach, a vast number of polymers, peptides and lipids containing histidine or imidazole have been designed as nucleic acid carriers (Midoux, P., C. Pichon, et al. (2009), Br I Pharmacol 157(2): 166-78). They are based on the fact that the imidazole ring functions as an endosome destabilizing factor which leads to the nucleic acid delivery into the cytosol. The imidazole ring of histidine is a weak base that has the ability to acquire a cationic charge when the pH of the environment drops below 6. As it has been demonstrated for poly(L-histidine) (a polypeptide consisting of multiple L-histidine) this phenomenon can induce membrane fusion and/or membrane permeation (of the endosome) in an acidic medium. Moreover, the accumulation of histidine residues inside acidic vesicles (endosomes) can induce a proton sponge effect, which increases osmolarity and their swelling. Therefore a carrier consisting at least partially of L-histidine can induce the disruption of endosomes. Upon review of all that prior art it appears that all described histidine-containing carriers of nucleic acids were complexed with the nucleic acid, which enabled the carrier as well the nucleic acid to be taken up together in one endosome.

In a further approach of the prior art L-histidine was utilized for the transfer of nucleic acids or genes. L-histidine is a well known pharmaceutical substance which is widely used in pharmaceutical formulations for different purposes e.g. as an inhibitor of free radical oxidation or as a stabilizer of secondary structures of proteins or antibodies. L-histidine is typically used as a component of the buffer system in high millimolar concentrations (generally 5-100 mM), which allows stabilizing the pH in a certain range (Wade, M.A., Tucker, H.N. (1998), J Nutr Biochem. 9, 308-315).

In this context, Evans et al. developed stable liquid formulations for adenovirus-based vaccines containing the free-radical oxidation inhibitors ethanol and histidine combined with the metal-ion chelator EDTA. The concentration of histidine applied for this purpose was 10 mM (Evans, R. K., D. K. Nawrocki, et al. (2004), J Pharm Sci 93(10): 2458-75). The high content of histidine in Evans et al. (2004, supra) led to a stabilisation of the pharmaceutically active ingredient in solution, however, no further impact on the contents of the liquid formulation was discussed. In this context, it is to be noted that in all prior art documents disclosing the use of free L-histidine as an additive for stabilization in pharmaceutical formulations the concentration is above 1 mM. However, even though a high histidine content as discussed in Evans et al. (2004, supra) may be advantageous in general, the present inventors found that (free) histidine in mmolar concentrations as used for the stabilization purpose is not effective to increase the transfection efficiency of a nucleic acid.

Summarizing the above, at present there is no disclosure in the art, which allows a skilled person to both efficiently transfect a nucleic acid (sequence) into a cell and to allow endosomal escape of said nucleic acid (sequence) without exhibiting a negative impact on translation of an encoded protein. In this context, a further still most challenging problem of the prior art represents the small amount of expressed protein or small biological activity of the nucleic acid obtained upon (inefficient) transfection into the cell or organism.

Accordingly, there is a long-lasting and urgent need in the art to provide means, which allow (a skilled person) to enhance transfection efficiency of nucleic acids especially of RNA for *in vitro* and particularly for *in vivo* applications, and which, as a further benefit, do not hinder or negatively influence endosomal release and translation of an encoded protein but rather improve same.

All these mentioned challenging problems are solved by the present invention, particularly by the subject matter of the attached claims. More precisely, the problem underlying the present invention is solved according to a first embodiment of the present invention by a solution for transfection and/or injection containing at least one nucleic acid (sequence) and (free) histidine in a concentration of about 1 to about 25 µM.

The present invention is particularly based on the unexpected finding that the addition of histidine in a low concentration of about 1 µM to about 25 µM in its non-complexed (free) form to the nucleic acid can efficiently improve the transfection efficiency of the nucleic acid. Application of such a solution containing the nucleic acid in vivo by injection leads to the distribution of the components of the solution in the tissue. Even though it is very unlikely that the nucleic acid and the free histidine are taken up by the same cell and more importantly by the same endosome, the present inventors surprisingly found that only a small amount of (free) histidine is sufficient to increase the transfection efficiency of the nucleic acid. Even more surprisingly, a higher amount of histidine does not lead to any improvement but rather to an impairment of the transfection.

The inventive solution of the underlying problem is particularly surprising and was not suggested by any of the above mentioned prior art documents. In contrast, reviewing the prior art a skilled person would have never suggested that - considering its teaching - addition of free histidine in a concentration of about 1 to about 25 µM could improve the transfection efficiency of a nucleic acid. As discussed above, in tissues it appears very unlikely that the nucleic acid and the histidine molecule enter the cell at the same time. Therefore, it was highly surprising for the present inventors to see that free histidine can in fact improve transfection efficiency *in vivo* as outlined herein. In contrast to the complex binding shown in the art the present inventors used free histidine which was only added to the solution and which was not complexed to the nucleic acid. As a combined uptake in a cell is unlikely due to the dilution effect in the tissue a skilled person would never have suggested that free histidine could improve transfection efficiency of nucleic acids, especially RNA. Additionally, it was absolutely unexpected by the present inventors that such a low concentration of (free) histidine of about 1 to about 25 µM furthermore improves the endosomal escape and thus leads to a better translation efficiency of the transfected nucleic acid in vivo.

According to the first embodiment of the present invention a solution for transfection and/or injection is provided containing at least one nucleic acid (sequence) and (free) histidine in a concentration of about 1 to about 25 µM. In this context "free" histidine is preferably understood as histidine, which is not contained in a carrier or complexation agent, a peptide or is not covalently bound to the nucleic acid (sequence).

In the context of the present invention, histidine is preferably L-histidine or D-histidine. Histidine as defined herein typically has the molecular formula C₆H₉N₃O₂. It is preferably identified under CAS number 71-00-1 (L-histidine) or 351-50-8 (D-histidine) and typically exhibits the following general structures:

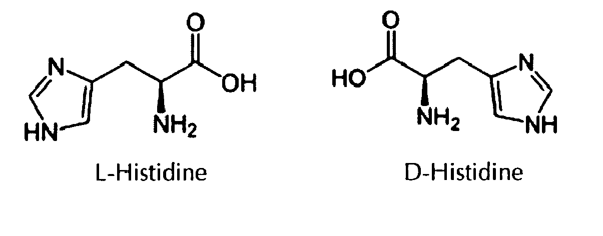

Histidine is also known under following synonymous terms:
- (2S)-2-Amino-3-(1H-imidazol-4-yl)propanoic acid
- (S)-4-(2-Amino-2-carboxyethyl)imidazole
- (S)-Histidine
- (S)-α-Amino-1H-imidazole-4-propanoic acid
- 1H-Imidazole-4-alanine, (S)-
- 1H-Imidazole-4-propanoic acid, α-amino-, (S)-
- Glyoxaline-5-alanine
- histidina
- L-Alanine, 3-(1H-imidazol-4-yl)-

In the context of the present invention, the term "histidine" further includes esters of histidine, such as L-histidine methyl ester or L-histidine ethyl ester (CAS 7555-06-8), and salts thereof, including L-histidine methyl ester dihydrochloride (CAS 7389-87-9).

According to the first embodiment, histidine, as used herein, is typically present in the inventive solution in a concentration of about 1 to about 25 µM, preferably in a concentration of about 5 to about 25 µM, more preferably in a concentration of about 10 to about 20 µM, even more preferably in a concentration of about 10 µM to about 15 µM, e.g. about 10 µM, about 11 µM, about 12 µM, about 13 µM, about 13 µM or about 15 µM, etc.

In the context of the present invention, the term "about" when used in combination with numerical values, refers to a range of numerical values in proximity to the specific numerical value listed. In particular, the term "about x" means "x ± 10%", more particularly "x ± 5%", and most particularly "x ± 1 %". In particular embodiments, the term "about x" refers to the range of values that represent the range determined by the measuring accuracy of the underlying experimental setting. When used in the context of numerical values that are integers only (such as for the number of nucleotides present in a nucleic acid sequence), the percentage values are rounded to the next integral value. However, in any such case of integral values, the term "about x" at least includes "x ± 1 ".

In the context of the present invention, the nucleic acid (sequence) as contained in the inventive solution for transfection and/or injection may be any suitable nucleic acid, selected e.g. from any (single-stranded or double-stranded) DNA, preferably, without being limited thereto, e.g. genomic DNA, single-stranded DNA molecules, double-stranded DNA molecules, coding DNA, DNA primers, DNA probes, immunostimulatory DNA, a (short) DNA oligonucleotide ((short) oligodesoxyribonucleotides), or may be selected e.g. from any PNA (peptide nucleic acid) or may be selected e.g. from any (single-stranded or double-stranded) RNA, preferably, without being limited thereto, a (short) RNA oligonucleotide ((short) oli-goribonucleotide), a coding RNA, a messenger RNA (mRNA), an immunostimulatory RNA, a siRNA, an antisense RNA, a micro RNA or riboswitches, ribozymes or aptamers; etc. The nucleic acid (sequence) of the inventive solution may also be a ribosomal RNA (rRNA), a transfer RNA (tRNA), a messenger RNA (mRNA), or a viral RNA (vRNA). Preferably, the nucleic acid (sequence) of the inventive solution is a RNA. More preferably, the nucleic acid (sequence) of the inventive solution is a (linear) single-stranded RNA, even more preferably a mRNA. In the context of the present invention, a mRNA is typically a RNA, which is composed of several structural elements, e.g. an optional 5'-UTR region, an upstream positioned ribosomal binding site followed by a coding region, an optional 3'-UTR region, which may be followed by a poly-A tail (and/or a poly-C-tail). A mRNA may occur as a mono-, di-, or even multicistronic RNA, i.e. a RNA which carries the coding sequences of one, two or more proteins or peptides. Such coding sequences in di-, or even multicistronic mRNA may be separated by at least one IRES sequence, e.g. as defined herein.

The nucleic acid (sequence) of the herein defined inventive solution for transfection and/or injection may be a single- or a double-stranded nucleic acid (sequence) (which may also be regarded as a nucleic acid (sequence) due to non-covalent association of two single-stranded nucleic acid(s) (sequences)) or a partially double-stranded or partially single stranded nucleic acid, which are at least partially self complementary (both of these partially double-stranded or partially single stranded nucleic acid sequences are typically formed by a longer and a shorter single-stranded nucleic acid sequence or by two single stranded nucleic acid sequences, which are about equal in length, wherein one single-stranded nucleic acid sequence is in part complementary to the other single-stranded nucleic acid sequence and both thus form a double-stranded nucleic acid sequence in this region, i.e. a partially double-stranded or partially single stranded nucleic acid sequence). Preferably, the nucleic acid (sequence) may be a single-stranded nucleic acid sequence. Furthermore, the nucleic acid (sequence) of the inventive solution may be a circular or linear nucleic acid molecule, preferably a linear nucleic acid molecule.

According to one alternative, the nucleic acid (sequence) of the herein defined inventive solution for transfection and/or injection may be a coding nucleic acid, e.g. a coding DNA or RNA. Such a coding DNA or RNA may be any DNA or RNA as defined herein. Preferably, such a coding DNA or RNA may be a single- or a double-stranded DNA or RNA, more preferably a single-stranded DNA or RNA, and/or a circular or linear DNA or RNA, more preferably a linear DNA or RNA. Even more preferably, the coding DNA or RNA may be a (linear) single-stranded DNA or RNA. Most preferably, the nucleic acid (sequence) of the herein defined inventive solution for transfection and/or injection may be a ((linear) single-stranded) messenger RNA (mRNA). Such a coding nucleic acid may occur as a mono-, di-, or even multicistronic RNA, i.e. a RNA which carries the coding sequences of one, two or more proteins or peptides. Such coding sequences in di-, or even multicistronic mRNAs may be separated by at least one IRES sequence.

Accordingly, the nucleic acid (sequence) of the herein defined inventive solution for transfection and/or injection may comprise (at least) one bi- or even multicistronic nucleic acid (sequences), preferably mRNA, i.e. (at least) one RNA which carries two or even more coding sequences of at least two (preferably different) peptides or proteins. Such coding sequences of the at least two (preferably different) peptides or proteins of the (at least) one bi-or even multicistronic nucleic acid (sequence) may be separated by at least one IRES (internal ribosomal entry site) sequence. In this context, a so-called IRES (internal ribosomal entry site) sequence can function as a sole ribosome binding site, but it can also serve to provide a bi- or even multicistronic nucleic acid (sequence) as defined herein which codes for several peptides or proteins, which are to be translated by the ribosomes independently of one another. Examples of IRES sequences which can be used according to the invention are those from picornaviruses (e.g. FMDV), pestiviruses (CFFV), polioviruses (PV), encephalo-myocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

### Coding nucleic acids:

The nucleic acid (sequence) of the herein defined inventive solution for transfection and/or injection may encode a protein or a peptide, which may be selected, without being restricted thereto, e.g. from therapeutically active proteins or peptides, including adjuvant proteins, from antigens, e.g. tumor antigens, pathogenic antigens (e.g. selected, from animal antigens, from viral antigens, from protozoal antigens, from bacterial antigens), allergenic antigens, autoimmune antigens, or further antigens, from allergens, from antibodies, from immunostimulatory proteins or peptides, from antigen-specific T-cell receptors, or from any other protein or peptide suitable for a specific (therapeutic) application, wherein the coding nucleic acid may be transported into a cell, a tissue or an organism and the protein may be expressed subsequently in this cell, tissue or organism.

### a) Therapeutically active proteins

In the context of the present invention, therapeutically active proteins may be encoded by the nucleic acid (sequence) of the herein defined inventive solution for transfection and/or injection. Therapeutically active proteins are defined herein as proteins which have an effect on healing, prevent prophylactically or treat therapeutically a disease, preferably as defined herein, or are proteins of which an individual is in need of. These may be selected from any naturally or synthetically designed occurring recombinant or isolated protein known to a skilled person from the prior art. Without being restricted thereto therapeutically active proteins may comprise proteins, capable of stimulating or inhibiting the signal transduction in the cell, e.g. cytokines, lymphokines, monokines, growth factors, receptors, signal transduction molecules, transcription factors, etc; anticoagulants; anti-thrombins; antiallergic proteins; apoptotic factors or apoptosis related proteins, therapeutic active enzymes and any protein connected with any acquired disease or any hereditary disease.

A therapeutically active protein, which may be encoded by the nucleic acid (sequence) of the herein defined inventive solution for transfection and/or injection, may also be an adjuvant protein. In this context, an adjuvant protein is preferably to be understood as any protein, which is capable to elicit an innate immune response as defined herein. Preferably, such an innate immune response comprises activation of a pattern recognition receptor, such as e.g. a receptor selected from the Toll-like receptor (TLR) family, including e.g. a Toll like receptor selected from human TLR1 to TLR10 or from murine Toll like receptors TLR1 to TLR13. More preferably, the adjuvant protein is selected from human adjuvant proteins or from pathogenic adjuvant proteins, selected from the group consisting of, without being limited thereto, bacterial proteins, protozoan proteins, viral proteins, or fungal proteins, animal proteins, in particular from bacterial adjuvant proteins. In addition, nucleic acids encoding human proteins involved in adjuvant effects may be used as well.

### b) Antigens

The nucleic acid (sequence) of the herein defined inventive solution for transfection and/or injection may alternatively encode an antigen. According to the present invention, the term "antigen" refers to a substance which is recognized by the immune system and is capable of triggering an antigen-specific immune response, e.g. by formation of antibodies or antigen-specific T-cells as part of an adaptive immune response. In this context, the first step of an adaptive immune response is the activation of naive antigen-specific T cells by antigen-presenting cells. This occurs in the lymphoid tissues and organs through which naïve T cells are constantly passing. The three cell types that can serve as antigen-presenting cells are dendritic cells, macrophages, and B cells. Each of these cells has a distinct function in eliciting immune responses. Tissue dendritic cells take up antigens by phagocytosis and macropinocytosis and are stimulated by infection to migrate to the local lymphoid tissue, where they differentiate into mature dendritic cells. Macrophages ingest particulate antigens such as bacteria and are induced by infectious agents to express MHC class II molecules. The unique ability of B cells to bind and internalize soluble protein antigens via their receptors may be important to induce T cells. By presenting the antigen on MHC molecules leads to activation of T cells which induces their proliferation and differentiation into armed effector T cells. The most important function of effector T cells is the killing of infected cells by CD8⁺ cytotoxic T cells and the activation of macrophages by TH1 cells which together make up cell-mediated immunity, and the activation of B cells by both TH2 and TH1 cells to produce different classes of antibody, thus driving the humoral immune response. T cells recognize an antigen by their T cell receptors which does not recognize and bind antigen directly, but instead recognize short peptide fragments e.g. of pathogens' protein antigens, which are bound to MHC molecules on the surfaces of other cells.

T cells fall into two major classes that have different effector functions. The two classes are distinguished by the expression of the cell-surface proteins CD4 and CD8. These two types of T cells differ in the class of MHC molecule that they recognize. There are two classes of MHC molecules - MHC class I and MHC class II molecules - which differ in their structure and expression pattern on tissues of the body. CD4⁺ T cells bind to a MHC class II molecule and CD8⁺ T cells to a MHC class I molecule. MHC class I and MHC class II molecules have distinct distributions among cells that reflect the different effector functions of the T cells that recognize them. MHC class I molecules present peptides from pathogens, commonly viruses to CD8⁺ T cells, which differentiate into cytotoxic T cells that are specialized to kill any cell that they specifically recognize. Almost all cells express MHC class I molecules, although the level of constitutive expression varies from one cell type to the next. But not only pathogenic peptides from viruses are presented by MHC class I molecules, also self-antigens like tumour antigens are presented by them. MHC class I molecules bind peptides from proteins degraded in the cytosol and transported in the endoplasmic reticulum. Thereby MHC class I molecules on the surface of cells infected with viruses or other cytosolic pathogens display peptides from these pathogen. The CD8⁺ T cells that recognize MHC class I:peptide complexes are specialized to kill any cells displaying foreign peptides and so rid the body of cells infected with viruses and other cytosolic pathogens. The main function of CD4⁺ T cells (CD4⁺ helper T cells) that recognize MHC class II molecules is to activate other effector cells of the immune system. Thus MHC class II molecules are normally found on B lymphocytes, dendritic cells, and macrophages, cells that participate in immune responses, but not on other tissue cells. Macrophages, for example, are activated to kill the intravesicular pathogens they harbour, and B cells to secrete immunoglobulins against foreign molecules. MHC class II molecules are prevented from binding to peptides in the endoplasmic reticulum and thus MHC class II molecules bind peptides from proteins which are degraded in endosomes. They can capture peptides from pathogens that have entered the vesicular system of macrophages, or from antigens internalized by immature dendritic cells or the immunoglobulin receptors of B cells. Pathogens that accumulate in large numbers inside macrophage and dendritic cell vesicles tend to stimulate the differentiation of TH1 cells, whereas extracellular antigens tend to stimulate the production of TH2 cells. TH1 cells activate the microbicidal properties of macrophages and induce B cells to make IgG antibodies that are very effective of opsonising extracellular pathogens for ingestion by phagocytic cells, whereas TH2 cells initiate the humoral response by activating naive B cells to secrete IgM, and induce the production of weakly opsonising antibodes such as IgG1 and IgG3 (mouse) and IgG2 and IgG4 (human) as well as IgA and IgE (mouse and human).

In the context of the present invention, antigens as encoded by the nucleic acid (sequence) of the herein defined inventive solution for transfection and/or injection typically comprise any antigen, falling under the above definition, more preferably protein and peptide antigens, e.g. tumor antigens, allergenic antigens, auto-immune self-antigens, pathogenic antigens, etc. In particular antigens as encoded by the nucleic acid (sequence) of the herein defined inventive solution for transfection and/or injection may be antigens generated outside the cell, more typically antigens not derived from the host organism (e.g. a human) itself (i.e. non-self antigens) but rather derived from host cells outside the host organism, e.g. viral antigens, bacterial antigens, fungal antigens, protozoological antigens, animal antigens, allergenic antigens, etc. Allergenic antigens (allergy antigens) are typically antigens, which cause an allergy in a human and may be derived from either a human or other sources. Additionally, antigens as encoded by the nucleic acid (sequence) of the herein defined inventive solution for transfection and/or injection may be furthermore antigens generated inside the cell, the tissue or the body. Such antigens include antigens derived from the host organism (e.g. a human) itself, e.g. tumor antigens, self-antigens or auto-antigens, such as auto-immune self-antigens, etc., but also (non-self) antigens as defined herein, which have been originally been derived from host cells outside the host organism, but which are fragmented or degraded inside the body, tissue or cell, e.g. by (protease) degradation, metabolism, etc.

One class of antigens as encoded by the nucleic acid (sequence) of the herein defined inventive solution for transfection and/or injection comprises tumor antigens. "Tumor antigens" are preferably located on the surface of the (tumor) cell. Tumor antigens may also be selected from proteins, which are overexpressed in tumor cells compared to a normal cell. Furthermore, tumor antigens also includes antigens expressed in cells which are (were) not themselves (or originally not themselves) degenerated but are associated with the supposed tumor. Antigens which are connected with tumor-supplying vessels or (re)formation thereof, in particular those antigens which are associated with neovascularization, e.g. growth factors, such as VEGF, bFGF etc., are also included herein. Antigens connected with a tumor furthermore include antigens from cells or tissues, typically embedding the tumor. Further, some substances (usually proteins or peptides) are expressed in patients suffering (knowingly or not-knowingly) from a cancer disease and they occur in increased concentrations in the body fluids of said patients. These substances are also referred to as "tumor antigens", however they are not antigens in the stringent meaning of an immune response inducing substance. The class of tumor antigens can be divided further into tumor-specific antigens (TSAs) and tumor-associated-antigens (TAAs). TSAs can only be presented by tumor cells and never by normal "healthy" cells. They typically result from a tumor specific mutation. TAAs, which are more common, are usually presented by both tumor and healthy cells. These antigens are recognized and the antigen-presenting cell can be destroyed by cytotoxic T cells. Additionally, tumor antigens can also occur on the surface of the tumor in the form of, e.g., a mutated receptor. In this case, they can be recognized by antibodies. Particular preferred tumor antigens are selected from the group consisting of 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R171, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGF, VEGFR-2/FLK-1, and WT1. Such tumor antigens preferably may be selected from the group consisting of MAGE-A1 (e.g. MAGE-A1 according to accession number M77481), MAGE-A2, MAGE-A3, MAGE-A6 (e.g. MAGE-A6 according to accession number NM_005363), MAGE-C1, MAGE-C2, melan-A (e.g. melan-A according to accession number NM_005511), GP100 (e.g. GP100 according to accession number M77348), tyrosinase (e.g. tyrosinase according to accession number NM_000372), surviving (e.g. survivin according to accession number AF077350), CEA (e.g. CEA according to accession number NM_004363), Her-2/neu (e.g. Her-2/neu according to accession number M11730), WT1 (e.g. WT1 according to accession number NM_000378), PRAME (e.g. PRAME according to accession number NM_006115), EGFRI (epidermal growth factor receptor 1) (e.g. EGFRI (epidermal growth factor receptor 1) according to accession number AF288738), MUC1, mucin-1 (e.g. mucin-1 according to accession number NM_002456), SEC61 G (e.g. SEC61 G according to accession number NM_014302), hTERT (e.g. hTERT accession number NM_198253), 5T4 (e.g. 5T4 according to accession number NM_006670), NY-Eso-1 (e.g. NY-Esol according to accession number NM_001327), TRP-2 (e.g. TRP-2 according to accession number NM_001922), STEAP, PCA, PSA, PSMA, etc.

According to another alternative, one further class of antigens as encoded by the nucleic acid (sequence) of the herein defined inventive solution for transfection and/or injection comprises allergenic antigens. Such allergenic antigens may be selected from antigens derived from different sources, e.g. from animals, plants, fungi, bacteria, etc. Allergens in this context include e.g. grasses, pollens, molds, drugs, or numerous environmental triggers, etc. Allergenic antigens typically belong to different classes of compounds, such as nucleic acids and their fragments, proteins or peptides and their fragments, carbohydrates, polysaccharides, sugars, lipids, phospholipids, etc. Of particular interest in the context of the present invention are antigens, which may be encoded by the nucleic acid (sequence) of the inventive solution, i.e. protein or peptide antigens and their fragments or epitopes, or nucleic acids and their fragments, particularly nucleic acids and their fragments, encoding such protein or peptide antigens and their fragments or epitopes.

### c) Antibodies

According to a further alternative, the nucleic acid (sequence) of the herein defined inventive solution for transfection and/or injection may encode an antibody or an antibody fragment. According to the present invention, such an antibody may be selected from any antibody, e.g. any recombinantly produced or naturally occurring antibodies, known in the art, in particular antibodies suitable for therapeutic, diagnostic or scientific purposes, or antibodies which have been identified in relation to specific cancer diseases. Herein, the term "antibody" is used in its broadest sense and specifically covers monoclonal and polyclonal antibodies (including agonist, antagonist, and blocking or neutralizing antibodies) and antibody species with polyepitopic specificity. According to the invention, the term "antibody" typically comprises any antibody known in the art (e.g. IgM, IgD, IgG, IgA and IgE antibodies), such as naturally occurring antibodies, antibodies generated by immunization in a host organism, antibodies which were isolated and identified from naturally occurring antibodies or antibodies generated by immunization in a host organism and recombinantly produced by biomolecular methods known in the art, as well as chimeric antibodies, human antibodies, humanized antibodies, bispecific antibodies, intrabodies, i.e. antibodies expressed in cells and optionally localized in specific cell compartments, and fragments and variants of the aforementioned antibodies. In general, an antibody consists of a light chain and a heavy chain both having variable and constant domains. The light chain consists of an N-terminal variable domain, V_{L}, and a C-terminal constant domain, C_{L}. In contrast, the heavy chain of the IgG antibody, for example, is comprised of an N-terminal variable domain, V_{H}, and three constant domains, C_{H}1, C_{H}2 und C_{H}3.

In the context of the present invention, antibodies as encoded by the nucleic acid (sequence) of the herein defined inventive solution for transfection and/or injection may preferably comprise full-length antibodies, i.e. antibodies composed of the full heavy and full light chains, as described above. However, derivatives of antibodies such as antibody fragments, variants or adducts may also be encoded by the nucleic acid (sequence) of the herein defined inventive solution for transfection and/or injection. Antibody fragments are preferably selected from Fab, Fab', F(ab')₂, Fc, Facb, pFc', Fd and Fv fragments of the aforementioned (full-length) antibodies. In general, antibody fragments are known in the art. For example, a Fab ("fragment, antigen binding") fragment is composed of one constant and one variable domain of each of the heavy and the light chain. The two variable domains bind the epitope on specific antigens. The two chains are connected via a disulfide linkage. A scFv ("single chain variable fragment") fragment, for example, typically consists of the variable domains of the light and heavy chains. The domains are linked by an artificial linkage, in general a polypeptide linkage such as a peptide composed of 15-25 glycine, proline and/or serine residues.

In the present context it is preferable that the different chains of the antibody or antibody fragment are encoded by a multicistronic nucleic acid (sequence). Alternatively, the different strains of the antibody or antibody fragment are encoded by several monocistronic nucleic acid(s) (sequences).

### dsRNA

According to a further alternative, the nucleic acid (sequence) of the herein defined inventive solution for transfection and/or injection may be in the form of dsRNA, preferably siRNA. A dsRNA, or a siRNA, is of interest particularly in connection with the phenomenon of RNA interference. The *in vitro* technique of RNA interference (RNAi) is based on double-stranded RNA molecules (dsRNA), which trigger the sequence-specific suppression of gene expression (Zamore (2001) Nat. Struct. Biol. 9: 746-750; Sharp (2001) Genes Dev. 5:485-490: Hannon (2002) Nature 41: 244-251). In the transfection of mammalian cells with long dsRNA, the activation of protein kinase R and RnaseL brings about unspecific effects, such as, for example, an interferon response (Stark et al. (1998) Annu. Rev. Biochem. 67: 227-264; He and Katze (2002) Viral Immunol. 15: 95-119). These unspecific effects are avoided when shorter, for example 21- to 23-mer, so-called siRNA (small interfering RNA), is used, because unspecific effects are not triggered by siRNA that is shorter than 30 bp (Elbashir et al. (2001) Nature 411: 494-498).

The nucleic acid (sequence) of the herein defined inventive solution for transfection and/or injection may thus be a double-stranded RNA (dsRNA) having a length of from 17 to 29, preferably from 19 to 25, and preferably being at least 90%, more preferably 95% and especially 100% (of the nucleotides of a dsRNA) complementary to a section of the nucleic acid (sequence) of a (therapeutically relevant) protein or antigen described (as active ingredient) hereinbefore or of any further protein as described herein, either a coding or a non-coding section, preferably a coding section. Such a (section of the) nucleic acid (sequence) may be termed herein a "target sequence" and may be any nucleic acid (sequence) as defined herein, preferably a genomic DNA, a cDNA, a RNA, e.g. a mRNA, etc. 90% complementary means that with a length of a dsRNA described herein of, for example, 20 nucleotides, this contains not more than 2 nucleotides without corresponding complementarity with the corresponding section of the target sequence. The sequence of the double-stranded RNA used according to the invention is, however, preferably wholly complementary in its general structure with a section of the target sequence. In this context the nucleic acid (sequence) of the inventive solution for transfection and/or injection may be a dsRNA having the general structure 5'-(N₁₇₋₂₉)-3', preferably having the general structure 5'-(N₁₉₋₂₅)-3', more preferably having the general structure 5'-(N₁₉₋₂₄)-3', or yet more preferably having the general structure 5'-(N₂₁₋₂₃)-3', wherein for each general structure each N is a (preferably different) nucleotide of a section of the target sequence, preferably being selected from a continuous number of 17 to 29 nucleotides of a section of the target sequence, and being present in the general structure 5'-(N₁₇₋₂₉)-3' in their natural order. In principle, all the sections having a length of from 17 to 29, preferably from 19 to 25, base pairs that occur in the target sequence can serve for preparation of a dsRNA as defined herein. Equally, dsRNAs used as nucleic acid (sequence) of the inventive solution for transfection and/or injection can also be directed against nucleotide sequences of a (therapeutically relevant) protein or antigen described (as active ingredient) hereinbefore that do not lie in the coding region, in particular in the 5' non-coding region of the target sequence, for example, therefore, against non-coding regions of the target sequence having a regulatory function. The target sequence of the dsRNA used as nucleic acid (sequence) of the solution for transfection and/or injection can therefore lie in the translated and untranslated region of the target sequence and/or in the region of the control elements of a protein or antigen described hereinbefore. The target sequence for a dsRNA used as the nucleic acid (sequence) of the inventive solution for transfection and/or injection can also lie in the overlapping region of untranslated and translated sequence; in particular, the target sequence can comprise at least one nucleotide upstream of the start triplet of the coding region, e.g. of a genomic DNA, a cDNA, a RNA, or a mRNA, etc.

According to another alternative, the nucleic acid (sequence) of the herein defined inventive solution for transfection and/or injection may be in the form of a(n) (immunostimulatory) CpG nucleic acid, in particular CpG-RNA or CpG-DNA. A CpG-RNA or CpG-DNA used according to the invention can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid used according to the invention is preferably in the form of CpG-RNA, more preferably in the form of single-stranded CpG-RNA (ss CpG-RNA). Also preferably, such CpG nucleic acids have a length as described above. Preferably the CpG motifs are unmethylated.

Likewise, according to a further alternative, the (immunostimulatory) nucleic acid (sequence) of the inventive solution for transfection and/or injection may be in the form of an immunostimulatory RNA, which preferably elicits an innate immune response. Such an immunostimulatory RNA used as the nucleic acid (sequence) of the inventive solution for transfection and/or injection may be any (double-stranded or single-stranded) RNA, e.g. a coding RNA, as defined herein. Preferably, the immunostimulatory RNA may be a single-stranded, a double-stranded or a partially double-stranded RNA, more preferably a single-stranded RNA, and/or a circular or linear RNA, more preferably a linear RNA. More preferably, the immunostimulatory RNA may be a (linear) single-stranded RNA. Even more preferably, the immunostimulatory RNA may be a ((linear) single-stranded) messenger RNA (mRNA). An immunostimulatory RNA may also occur as a short RNA oligonucleotide as defined herein. An immunostimulatory RNA as used herein may furthermore be selected from any class of RNA molecules, found in nature or being prepared synthetically, and which can induce an (innate) immune response. In this context, an immune response may occur in various ways. A substantial factor for a suitable immune response is the stimulation of different T-cell sub-populations. T-lymphocytes are typically divided into two sub-populations, the T-helper 1 (Th1) cells and the T-helper 2 (Th2) cells, with which the immune system is capable of destroying intracellular (Th1) and extracellular (Th2) pathogens (e.g. antigens). The two Th cell populations differ in the pattern of the effector proteins (cytokines) produced by them. Thus, Th1 cells assist the cellular immune response by activation of macrophages and cytotoxic T-cells. Th2 cells, on the other hand, promote the humoral immune response by stimulation of the B-cells for conversion into plasma cells and by formation of antibodies (e.g. against antigens). The Th1/Th2 ratio is therefore of great importance in the immune response. In connection with the present invention, the Th1/Th2 ratio of the immune response is preferably shifted in the direction towards the cellular response (Th1 response) and a cellular immune response is thereby induced. According to one example, the immune system may be activated by ligands of Toll-like receptors (TLRs). TLRs are a family of highly conserved pattern recognition receptor (PRR) polypeptides that recognize pathogen-associated molecular patterns (PAMPs) and play a critical role in innate immunity in mammals. Currently at least thirteen family members, designated TLR1 - TLR13 (Toll-like receptors: TLR1, TLR2, TLR3, TLR4, TLR5, TLR6, TLR7, TLR8, TLR9, TLR10, TLR11, TLR12 or TLR13), have been identified. Furthermore, a number of specific TLR ligands have been identified. It was e.g. found that unmethylated bacterial DNA and synthetic analogs thereof (CpG DNA) are ligands for TLR9 (Hemmi H et al. (2000) Nature 408:740-5; Bauer S et al. (2001) Proc NatlAcadSci USA 98, 9237-42). Furthermore, it has been reported that ligands for certain TLRs include certain nucleic acid molecules and that certain types of RNA are immunostimulatory in a sequence-independent or sequence-dependent manner, wherein these various immunostimulatory RNAs may e.g. stimulate TLR3, TLR7, or TLR8, or intracellular receptors such as RIG-I, MDA-5, etc. E.g. Lipford *et al.* determined certain G,U-containing oligoribonucleotides as immunostimulatory by acting via TLR7 and TLR8 (see WO 03/086280). The immunostimulatory G,U-containing oligoribonucleotides described by Lipford *et al.* were believed to be derivable from RNA sources including ribosomal RNA, transfer RNA, messenger RNA, and viral RNA.

The immunostimulatory RNA used as the nucleic acid (sequence) of the herein defined inventive solution for transfection and/or injection may thus comprise any RNA sequence known to be immunostimulatory, including, without being limited thereto, RNA sequences representing and/or encoding ligands of TLRs, preferably selected from family members TLR1 - TLR13, more preferably from TLR7 and TLR8, ligands for intracellular receptors for RNA (such as RIG-I or MAD-5, etc.) (see e.g. Meylan, E., Tschopp, J. (2006). Toll-like receptors and RNA helicases: two parallel ways to trigger antiviral responses. Mol. Cell 22, 561-569), or any other immunostimulatory RNA sequence. Furthermore, (classes of) immunostimulatory RNA molecules, used as the nucleic acid (sequence) of the inventive solution for transfection and/or injection may include any other RNA capable of eliciting an immune response. Without being limited thereto, such an immunostimulatory RNA may include ribosomal RNA (rRNA), transfer RNA (tRNA), messenger RNA (mRNA), and viral RNA (vRNA).

In a further preferred embodiment the nucleic acid (sequence) of the herein defined inventive solution for transfection and/or injection as defined herein may also occur in the form of a modified nucleic acid.

According to a first aspect, the nucleic acid (sequence) of the herein defined inventive solution for transfection and/or injection may thus be provided as a "stabilized nucleic acid", preferably as a stabilized RNA, more preferably as a RNA that is essentially resistant to *in vivo* degradation (e.g. by an exo- or endo-nuclease).

In this context, the nucleic acid (sequence) of the herein defined inventive solution for transfection and/or injection may contain backbone modifications, sugar modifications or base modifications. A backbone modification in connection with the present invention is a modification in which phosphates of the backbone of the nucleotides contained in the nucleic acid (sequence) of the inventive solution for transfection and/or injection are chemically modified. A sugar modification in connection with the present invention is a chemical modification of the sugar of the nucleotides of the nucleic acid (sequence) of the inventive solution for transfection and/or injection. Furthermore, a base modification in connection with the present invention is a chemical modification of the base moiety of the nucleotides of the nucleic acid (sequence) of the inventive solution for transfection and/or injection.

According to a further aspect, the nucleic acid (sequence) of the herein defined inventive solution for transfection and/or injection can contain a lipid modification. Such a lipid-modified nucleic acid typically comprises a nucleic acid as defined herein. Such a lipid-modified nucleic acid (sequence) of the inventive solution for transfection and/or injection typically further comprises at least one linker covalently linked with that nucleic acid (sequence), and at least one lipid covalently linked with the respective linker. Alternatively, the lipid-modified nucleic acid (sequence) comprises an at least one nucleic acid (sequence) as defined herein and at least one (bifunctional) lipid covalently linked (without a linker) with that nucleic acid (sequence). According to a third alternative, the lipid-modified nucleic acid (sequence) comprises a nucleic acid (sequence) as defined herein, at least one linker covalently linked with that nucleic acid (sequence), and at least one lipid covalently linked with the respective linker, and also at least one (bifunctional) lipid covalently linked (without a linker) with that nucleic acid (sequence).

The nucleic acid (sequence) of the inventive solution for transfection and/or injection may likewise be stabilized in order to prevent degradation of the nucleic acid by various approaches, particularly, when RNA or mRNA is used as a nucleic acid (sequence) for the inventive purpose. It is known in the art that instability and (fast) degradation of mRNA or of RNA in general may represent a serious problem in the application of RNA based compositions. This instability of RNA is typically due to RNA-degrading enzymes, "RNAases" (ribonucleases), wherein contamination with such ribonucleases may sometimes completely degrade RNA in solution. Accordingly, the natural degradation of mRNA in the cytoplasm of cells is very finely regulated and RNase contaminations may be generally removed by special treatment prior to use of said compositions, in particular with diethyl pyrocarbonate (DEPC). A number of mechanisms of natural degradation are known in this connection in the prior art, which may be utilized as well. E.g., the terminal structure is typically of critical importance for a mRNA. As an example, at the 5' end of naturally occurring mRNAs there is usually a so-called "cap structure" (a modified guanosine nucleotide), and at the 3' end is typically a sequence of up to 200 adenosine nucleotides (the so-called poly-A tail).

According to another aspect, the nucleic acid (sequence) of the herein defined inventive solution for transfection and/or injection may be modified, and thus stabilized, especially if the nucleic acid (sequence) is in the form of a mRNA, by modifying the G/C content of the nucleic acid (sequence), particularly a mRNA, preferably of the coding region thereof.

In a particularly preferred aspect of the present invention, the G/C content of the coding region of the nucleic acid (sequence) of the herein defined inventive solution for transfection and/or injection, especially if the nucleic acid (sequence) is in the form of a mRNA, is modified, particularly increased, compared to the G/C content of the coding region of its particular wild type mRNA, i.e. the unmodified mRNA. The encoded amino acid sequence of the at least one mRNA is preferably not modified compared to the coded amino acid sequence of the particular wild type mRNA.

This modification of the nucleic acid (sequence) of the herein defined inventive solution for transfection and/or injection, especially if the nucleic acid (sequence) is in the form of a mRNA or codes for a mRNA, is based on the fact that the sequence of any mRNA region to be translated is important for efficient translation of that mRNA. Thus, the composition and the sequence of various nucleotides are important. In particular, sequences having an increased G (guanosine)/C (cytosine) content are more stable than sequences having an increased A (adenosine)/U (uracil) content. According to the invention, the codons of the mRNA are therefore varied compared to its wild type mRNA, while retaining the translated amino acid sequence, such that they include an increased amount of G/C nucleotides. In respect to the fact that several codons code for one and the same amino acid (so-called degeneration of the genetic code), the most favorable codons for the stability can be determined (so-called alternative codon usage).

Depending on the amino acid to be encoded by the nucleic acid (sequence) of the herein defined inventive solution for transfection and/or injection, especially if the nucleic acid is in the form of a mRNA or codes for a mRNA, there are various possibilities for modification of the at least one mRNA sequence, compared to its wild type sequence. In the case of amino acids which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U is present.

In contrast, codons which contain A and/or U nucleotides can be modified by substitution of other codons which code for the same amino acids but contain no A and/or U. Examples of these are:
the codons for Pro can be modified from CCU or CCA to CCC or CCG;
the codons for Arg can be modified from CGU or CGA or AGA or AGG to CGC or CGG;
the codons for Ala can be modified from GCU or GCA to GCC or GCG;
the codons for Gly can be modified from GGU or GGA to GGC or GGG.

In other cases, although A or U nucleotides cannot be eliminated from the codons, it is however possible to decrease the A and U content by using codons which contain a lower content of A and/or U nucleotides. Examples of these are:
the codons for Phe can be modified from UUU to UUC;
the codons for Leu can be modified from UUA, UUG, CUU or CUA to CUC or CUG;
the codons for Ser can be modified from UCU or UCA or AGU to UCC, UCG or AGC;
the codon for Tyr can be modified from UAU to UAC;
the codon for Cys can be modified from UGU to UGC;
the codon for His can be modified from CAU to CAC;
the codon for Gln can be modified from CAA to CAG;
the codons for Ile can be modified from AUU or AUA to AUC;
the codons for Thr can be modified from ACU or ACA to ACC or ACG;
the codon for Asn can be modified from AAU to AAC;
the codon for Lys can be modified from AAA to AAG;
the codons for Val can be modified from GUU or GUA to GUC or GUG;
the codon for Asp can be modified from GAU to GAC;
the codon for Glu can be modified from GAA to GAG;
the stop codon UAA can be modified to UAG or UGA.

In the case of the codons for Met (AUG) and Trp (UGG), on the other hand, there is no possibility of sequence modification.

The substitutions listed above can be used either individually or in all possible combinations to increase the G/C content of the nucleic acid (sequence) of the herein defined inventive solution for transfection and/or injection, especially if the nucleic acid is in the form of a mRNA or codes for a mRNA, compared to its particular wild type mRNA (i.e. the original sequence). Thus, for example, all codons for Thr occurring in the wild type sequence can be modified to ACC (or ACG).

Preferably, the G/C content of the coding region of the nucleic acid (sequence) of the herein defined inventive solution for transfection and/or injection, especially if the nucleic acid is in the form of a mRNA or codes for a mRNA, is increased by at least 7%, more preferably by at least 15%, particularly preferably by at least 20%, compared to the G/C content of the coded region of the wild type mRNA. According to a specific aspect at least 5%, 10%, 20%, 30%, 40%, 50%, 60%, more preferably at least 70 %, even more preferably at least 80% and most preferably at least 90%, 95% or even 100% of the substitutable codons in the region coding for a protein or peptide as defined herein or its fragment or variant thereof or the whole sequence of the wild type mRNA sequence are substituted, thereby increasing the GC/content of said sequence.

In this context, it is particularly preferable to increase the G/C content of the nucleic acid (sequence) of the herein defined inventive solution for transfection and/or injection, especially if the nucleic acid is in the form of a mRNA or codes for a mRNA, to the maximum (i.e. 100% of the substitutable codons), in particular in the region coding for a protein, compared to the wild type sequence.

According to the invention, a further preferred modification of the nucleic acid (sequence) of the herein defined inventive solution for transfection and/or injection, especially if the nucleic acid is in the form of a mRNA or codes for a mRNA, is based on the finding that the translation efficiency is also determined by a different frequency in the occurrence of tRNAs in cells. Thus, if so-called "rare codons" are present in the nucleic acid (sequence) of the inventive solution for transfection and/or injection, especially if the nucleic acid is in the form of a mRNA or codes for a mRNA, to an increased extent, the corresponding modified nucleic acid (sequence) is translated to a significantly poorer degree than in the case where codons coding for relatively "frequent" tRNAs are present.

Especially if the modified nucleic acid (sequence) of the herein defined inventive solution for transfection and/or injection is in the form of a mRNA or codes for a mRNA, the coding region of the modified nucleic acid is preferably modified compared to the corresponding region of the wild type mRNA such that at least one codon of the wild type sequence which codes for a tRNA which is relatively rare in the cell is exchanged for a codon which codes for a tRNA which is relatively frequent in the cell and carries the same amino acid as the relatively rare tRNA. By this modification, the sequences of the nucleic acid (sequence) of the inventive solution for transfection and/or injection, especially if the nucleic acid is in the form of a mRNA or codes for a mRNA, is modified such that codons for which frequently occurring tRNAs are available are inserted. In other words, according to the invention, by this modification all codons of the wild type sequence which code for a tRNA which is relatively rare in the cell can in each case be exchanged for a codon which codes for a tRNA which is relatively frequent in the cell and which, in each case, carries the same amino acid as the relatively rare tRNA.

Which tRNAs occur relatively frequently in the cell and which, in contrast, occur relatively rarely is known to a person skilled in the art; cf. e.g. Akashi, Curr. Opin. Genet. Dev. 2001, 11 (6): 660-666. The codons which use for the particular amino acid the tRNA which occurs the most frequently, e.g. the Gly codon, which uses the tRNA which occurs the most frequently in the (human) cell, are particularly preferred.

According to the invention, it is particularly preferable to link the sequential G/C content which is increased, in particular maximized, in the modified nucleic acid (sequence) of the herein defined inventive solution for transfection and/or injection, especially if the nucleic acid is in the form of a mRNA or codes for a mRNA, with the "frequent" codons without modifying the amino acid sequence of the protein encoded by the coding region of the nucleic acid (sequence). This preferred aspect allows provision of a particularly efficiently translated and stabilized (modified) nucleic acid (sequence) of the inventive solution for transfection and/or injection, especially if the nucleic acid is in the form of a mRNA or codes for a mRNA.

According to a further preferred aspect of the invention, the nucleic acid (sequence) of the inventive solution for transfection and/or injection as defined herein, especially if the nucleic acid is in the form of a coding nucleic acid (sequence), furthermore preferably has at least one 5' and/or 3' stabilizing sequence. These stabilizing sequences in the 5' and/or 3' untranslated regions have the effect of increasing the half-life of the nucleic acid in the cytosol. These stabilizing sequences can have 100% sequence identity to naturally occurring sequences which occur in viruses, bacteria and eukaryotes, but can also be partly or completely synthetic. The untranslated sequences (UTR) of the (alpha-)globin gene, e.g. from *Homo sapiens* or *Xenopus laevis* may be mentioned as an example of stabilizing sequences which can be used in the present invention for a stabilized nucleic acid. Another example of a stabilizing sequence has the general formula (C/U)CCANₓCCC(U/A)PyₓUC(C/U)CC (SEQ ID NO: 4), which is contained in the 3'UTR of the very stable RNA which codes for (alpha-)globin, type(I)-collagen, 15-lipoxygenase or for tyrosine hydroxylase (cf. Holcik et al., Proc. Natl. Acad. Sci. USA 1997, 94: 2410 to 2414). Such stabilizing sequences can of course be used individually or in combination with one another and also in combination with other stabilizing sequences known to a person skilled in the art.

Nevertheless, substitutions, additions or eliminations of bases are preferably carried out with the nucleic acid (sequence) of the inventive solution for transfection and/or injection as defined herein, especially if the nucleic acid is in the form of a mRNA, using a DNA matrix for preparation of the nucleic acid by techniques of the well known site directed mutagenesis or with an oligonucleotide ligation strategy (see e.g. Maniatis et al., Molecular Cloning: A Laboratory Manual, Cold Spring Harbor Laboratory Press, 3rd ed., Cold Spring Harbor, NY, 2001). In such a process, for preparation of the nucleic acid (sequence) of the inventive solution for transfection and/or injection as defined herein, especially if the nucleic acid is in the form of a mRNA, a corresponding DNA molecule may be transcribed *in vitro.* This DNA matrix preferably comprises a suitable promoter, e.g. a T7 or SP6 promoter, for *in vitro* transcription, which is followed by the desired nucleotide sequence for the nucleic acid, e.g. mRNA, to be prepared and a termination signal for *in vitro* transcription. The DNA molecule, which forms the matrix of at least one RNA of interest, may be prepared by fermentative proliferation and subsequent isolation as part of a plasmid which can be replicated in bacteria. Plasmids which may be mentioned as suitable for the present invention are e.g. the plasmids pT7Ts (GenBank accession number U26404; Lai et al., Development 1995, 121: 2349 to 2360), pGEM^{®} series, e.g. pGEM^{®}-1 (GenBank accession number X65300; from Promega) and pSP64 (GenBank accession number X65327); cf. also Mezei and Storts, Purification of PCR Products, in: Griffin and Griffin (ed.), PCR Technology: Current Innovation, CRC Press, Boca Raton, FL, 2001.

Nucleic acid molecules used according to the invention as defined herein may be prepared using any method known in the art, including synthetic methods such as e.g. solid phase synthesis, as well as *in vitro* methods, such as *in vitro* transcription reactions.

According to another particularly preferred aspect, the nucleic acid (sequence) of the inventive solution for transfection and/or injection as defined herein, especially if the nucleic acid is in the form of a coding nucleic acid (sequence) may additionally or alternatively encode a secretory signal peptide. Such signal peptides are sequences, which typically exhibit a length of about 15 to 30 amino acids and are preferably located at the N-terminus of the encoded peptide, without being limited thereto. Signal peptides as defined herein preferably allow the transport of the protein or peptide as encoded by the nucleic acid (sequence) of the inventive solution for transfection and/or injection as defined herein, especially if the nucleic acid is in the form of a mRNA, into a defined cellular compartiment, preferably the cell surface, the endoplasmic reticulum (ER) or the endosomal-lysosomal compartiment. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulines as defined herein, signal sequences of the invariant chain of immunoglobulines or antibodies as defined herein, signal sequences of Lamp1, Tapasin, Erp57, Calretikulin, Calnexin, and further membrane associated proteins or of proteins associated with the endoplasmic reticulum (ER) or the endosomal-lysosomal compartiment. Particularly preferably, signal sequences of MHC class I molecule HLA-A*0201 may be used according to the present invention.

Any of the above modifications may be applied to the nucleic acid (sequence) of the inventive solution for transfection and/or injection as defined herein, especially if the nucleic acid is in the form of a mRNA, and further to any nucleic acid as used in the context of the present invention and may be, if suitable or necessary, be combined with each other in any combination, provided, these combinations of modifications do not interfere with each other in the respective nucleic acid. A person skilled in the art will be able to take his choice accordingly.

The nucleic acid (sequence) of the inventive solution for transfection and/or injection as defined herein as well as proteins or peptides as encoded by this nucleic acid (sequence) may comprise fragments or variants of those sequences. Such fragments or variants may typically comprise a sequence having a sequence identity with one of the above mentioned nucleic acids, or with one of the proteins or peptides or sequences, if encoded by the at least one nucleic acid (sequence) of at least 5%, 10%, 20%, 30%, 40%, 50%, 60%, preferably at least 70%, more preferably at least 80%, equally more preferably at least 85%, even more preferably at least 90% and most preferably at least 95% or even 97%, to the entire wild type sequence, either on nucleic acid level or on amino acid level.

"Fragments" of proteins or peptides in the context of the present invention (encoded by a nucleic acid as defined herein) may comprise a sequence of a protein or peptide as defined herein, which is, with regard to its amino acid sequence (or its encoded nucleic acid (sequence)), N-terminally, C-terminally and/or intrasequentially truncated compared to the amino acid sequence of the original (native) protein (or its encoded nucleic acid (sequence)). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid (sequence) of such a protein or peptide. Likewise, "fragments" of nucleic acids in the context of the present invention may comprise a sequence of a nucleic acid as defined herein, which is, with regard to its nucleic acid (sequence) 5'-, 3'-and/or intrasequentially truncated compared to the nucleic acid (sequence) of the original (native) nucleic acid (sequence). A sequence identity with respect to such a fragment as defined herein may therefore preferably refer to the entire nucleic acid as defined herein.

Fragments of proteins or peptides in the context of the present invention (encoded by the nucleic acid (sequence) of the inventive solution for transfection and/or injection as defined herein) may furthermore comprise a sequence of a protein or peptide as defined herein, which has a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form.

Fragments of proteins or peptides as defined herein (encoded by the nucleic acid (sequence) of the inventive solution for transfection and/or injection as defined herein may also comprise epitopes of those proteins or peptides. Epitopes (also called "antigen determinants") in the context of the present invention are typically fragments located on the outer surface of (native) proteins or peptides as defined herein, preferably having 5 to 15 amino acids, more preferably having 5 to 12 amino acids, even more preferably having 6 to 9 amino acids, which may be recognized by antibodies or B-cell receptors, i.e. in their native form. Such epitopes of proteins or peptides may furthermore be selected from any of the herein mentioned variants of such proteins or peptides. In this context antigenic determinants can be conformational or discontinous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

"Variants" of proteins or peptides as defined herein may be encoded by the nucleic acid (sequence) of the inventive solution for transfection and/or injection as defined herein, wherein nucleic acids of the nucleic acid, encoding the protein or peptide as defined herein, are exchanged. Thereby, a protein or peptide may be generated, having an amino acid sequence which differs from the original sequence in one or more mutation(s), such as one or more substituted, inserted and/or deleted amino acid(s). Preferably, these fragments and/or variants have the same biological function or specific activity compared to the full-length native potein, e.g. its specific antigenic property.

The nucleic acid (sequence) of the inventive solution for transfection and/or injection as defined herein may also encode a protein or peptide as defined herein, wherein the encoded amino acid sequence comprises conservative amino acid substitution(s) compared to its physiological sequence. Those encoded amino acid sequences as well as their encoding nucleotide sequences in particular fall under the term variants as defined herein. Substitutions in which amino acids which originate from the same class are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

Furthermore, variants of proteins or peptides as defined herein, which may be encoded by the nucleic acid (sequence) of the inventive solution for transfection and/or injection as defined herein, may also comprise those sequences, wherein nucleotides of the nucleic acid are exchanged according to the degeneration of the genetic code, without leading to an alteration of the respective amino acid sequence of the protein or peptide, i.e. the amino acid sequence or at least part thereof may not differ from the original sequence in one or more mutation(s) within the above meaning.

In order to determine the percentage to which two sequences, e.g. nucleic acid(s) (sequences) as defined herein, preferably the amino acid sequences encoded by nucleic acid(s) (sequences) as defined herein, e.g. of the proteins or peptides as defined herein), or the amino acid sequences them selves, are identical, the sequences can be aligned in order to be subsequently compared to one another. Therefore, e.g. gaps can be inserted into the sequence of the first sequence and the component at the corresponding position of the second sequence can be compared. If a position in the first sequence is occupied by the same component as is the case at a position in the second sequence, the two sequences are identical at this position. The percentage to which two sequences are identical is a function of the number of identical positions divided by the total number of positions. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program.

According to a further preferred aspect the inventive solution for transfection and/or injection as defined herein, containing at least one nucleic acid (sequence) as defined herein and (free) histidine, may additionally contain a lactate. Such a lactate provides a surprisingly good effect on stabilization of the nucleic acid (sequence) of the inventive solution for transfection and/or injection during lyophilization.

A lactate as defined herein may be any lactate available in the art. Preferably, a lactate within the context of the present invention is defined as a chemical compound, particularly a salt, derived from free lactic acid (IUPAC systematic name: 2-hydroxypropanoic acid), also known as milk acid, including its optical isomers L-(+)-lactic acid, (*S*)-lactic acid, D-(-)-lactic acid or (*R*)-lactic acid, more preferably its biologically active optical isomer L-(+)-lactic acid, wherein the salt or an anion thereof, preferably may be selected from sodium-lactate, potassium-lactate, or Al₃⁺-lactate, NH₄⁺-lactate, Fe-lactate, Li-lactate, Mg-lactate, Ca-lactate, Mn-lactate or Ag-lactate, or selected from Ringer's lactate (RiLa), lactated Ringer's solution (main content sodium lactate, also termed "Hartmann's Solution" in the UK), acetated Ringer's solution, or selected from lactate containing water, or ortho-lactate-containing (isotonic) solutions (e.g. for injection purposes), etc.

The inventive solution for transfection and/or injection as defined herein may typically comprise a lactate concentration in the range of about 3 mM to about 300 mM, preferably in the range of about 5 mM to about 200 mM, more preferably in the range of about 10 mM to about 150 mM, even more preferably about 15 mM to about 35 mM, and most preferably 20 mM to about 31 mM.

Alternatively, the inventive solution for transfection and/or injection as defined herein may typically comprise a Ringer's lactate content (or a content of any of the aforementioned (undiluted) lactate containing solutions) e.g. in the range of about 10% (w/w) to about 100% (w/w), e.g. in the range of about 20% (w/w) to about 100% (w/w), in the range of about 30% (w/w) to about 100% (w/w), in the range of about 40% (w/w) to about 100% (w/w), in the range of about 50% (w/w) to about 90% (w/w), preferably in the range of about 60% (w/w) to about 90% (w/w), more preferably in the range of about 70% (w/w) to about 90% (w/w), e.g. about 80% (w/w), of Ringer's lactate (or the aforementioned (undiluted) lactate containing solution). In this context, Ringer's lactate (100 % (w/w)) is typically defined as a solution comprising 131 mM Na⁺, 5.36 mM K⁺, 1.84 mM Ca²⁺, and 28.3 mM lactate).

In a further preferable aspect, the herein defined inventive solution for transfection and/or injection containing at least one nucleic acid (sequence) and (free) histidine additionally contains mannose which stabilizes the at least one nucleic acid (sequence) contained in the inventive solution for transfection and/or injection during lyophilization and/or improves biological activity of the nucleic acid (sequence). In the context of the present invention, mannose is preferably a sugar monomer of the aldohexose series of carbohydrates. Mannose as defined herein typically has the molecular formula C₆H₁₂C₆, is also known under its IUPAC nomenclature as (2*S*,3*S*,4*R*,5*R*)-Pentahydroxyhexanal, (2*R*,3*R*,4*S*,5*S*)-Pentahydroxyhexanal. It is preferably identified under CAS number 31103-86-3.

According to a more particular aspect, mannose as used herein is typically present in the herein defined inventive solution for transfection and/or injection in a concentration of about 0.01 to about 10% (w/w), preferably in a concentration of about 0.01 to about 10% (w/w), more preferably in a concentration of about 0.1 to about 7.5% (w/w), even,more preferably in a concentration of about 0.5 to about 5% (w/w), and most preferably in a concentration of about 1 to about 4% (w/w), e.g. a concentration of about 2 to about 4% (w/w), such as about 2.5 % (w/w). Herein, a concentration of about 1% (w/w) mannose corresponds to a concentration of about 55 mM mannose. Any of the above and herein mentioned values and concentrations for mannose in % (w/w) may thus be calculated in mM on the above basis.

The inventive solution for transfection and/or injection as defined herein, containing at least one nucleic acid (sequence) and (free) histidine, may additionally contain water, preferably water for injection (WFI).

The inventive solution for transfection and/or injection as defined herein, containing at least one nucleic acid (sequence) and (free) histidine, may additionally contain further optional components or additives, e.g. a cryoprotectant, a lyoprotectant or any further suitable additive, preferably as defined in the following.

Preferably, the herein defined inventive solution for transfection and/or injection may contain the herein defined contents, optional components, additives, etc. in such a concentration so as to lead to an osmolality comparable to that of blood plasma. In this context, the term "osmolality" is typically to be understood as a measure of all contents, optional components, additives, etc. of the inventive solution for transfection and/or injection as defined herein. Osmolality is typically the measure of solute concentration, defined as the number of osmoles (Osm) of all solubilized contents, optional components, additives, etc. per liter (I) of solution (osmol/l or osm/l). In the present context, the inventive solution for transfection and/or injection as defined herein may comprise an osmolality preferably in the range of about 200 mosmol/l to about 400 mosmol/l, more preferably in the range of about 250 mosmol/I to about 350 mosmol/l, even more preferably in the range of about 270 mosmol/l to about 330 mosmol/l or in the range of about 280 mosmol/l to about 320 mosmol/l, or in the range of about e.g. about 290 mosmol/l to about 310 mosmol/l, e.g. about 295 mos-mol/l, about mosmol/l, about 296 mosmol/l, about 297 mosmol/l, about 298 mosmol/l, about 299 mosmol/l, about, 300 mosmol/l, about 301 mosmol/l, about 302 mosmol/l, about 303 mosmol/l, about 304 mosmol/l, about 305 mosmol/l, about 306 mosmol/l, about 307 mosmol/l, about 308 mosmol/l.

As a particularly preferred optional component or additive, the herein defined inventive solution for transfection and/or injection may additionally contain at least one suspending agent, preferably mannit, preferably in a concentration of about 1 to 15% (w/w), more preferably in a concentration of about 3 to 10% (w/w), and even more preferably in a concentration of about 4 to 6% (w/w).

As a further component, the herein defined inventive solution for transfection and/or injection may additionally contain at least one optional component or additive selected, e.g., from mannite, proteins, peptides, amino acids, alcohols, carbohydrates, metals or metal ions, surfactants, polymers or complexing agents, buffers, etc., or a combination thereof.

In the context of the present invention, another optional component or additive of the herein defined inventive solution for transfection and/or injection may also be selected from the group of amino acids other than histidine. Such group may comprise, without being limited thereto, any naturally occurring amino acid other than histidine. Cryoprotectants and/or lyoprotectants selected from the group of amino acids may additionally comprise any modification of a naturally occurring amino acid other than histidine.

Furthermore, in the context of the inventive solution for transfection and/or injection as defined herein, a further optional component or additive may be selected from the group of alcohols. Such group may comprise, without being limited thereto, any alcohol suitable for the preparation of a pharmaceutical composition, preferably, without being limited thereto, mannitol, polyethyleneglycol, polypropyleneglycol, sorbitol, etc.

Additionally, in the context of the inventive solution for transfection and/or injection as defined herein, a further optional component or additive may be selected from the group of carbohydrates. Such group of carbohydrates may comprise, without being limited thereto, any carbohydrate, suitable for the preparation of a pharmaceutical composition, preferably, without being limited thereto, monosaccharides, such as e.g. glucose, fructose, etc., disaccharides, such as e.g. lactose, maltose, sucrose, trehalose, etc., and polysaccharides, such as e.g. dextran, HP-beta CD, etc.

Also, in the context of the inventive solution for transfection and/or injection as defined herein, a further suitable optional component or additive may be selected from the group of proteins. Such group may comprise, without being limited thereto, proteins such as albumin, gelatine, therapeutically active proteins as defined herein, antibodies as defined herein, antigens as defined herein, or any further protein as defined herein.

A further optional component or additive, which may be contained in the inventive solution for transfection and/or injection as defined herein may be selected from the group of metals or metal ions, typically comprising, without being limited thereto, metals or metal ions or salts, e.g. selected from alkali metals, alkaline earth metals, transition metals, earth metals or members of the boron group, metalloids or semi metals.

In the context of the present invention, a further optional component or additive of the inventive solution for transfection and/or injection as defined herein may be selected from the group of surfactants comprising, without being limited thereto, any surfactant, suitable for the preparation of a pharmaceutical composition, preferably, without being limited thereto, Tween, e.g. Tween 80 (e.g. 0.2%), Pluronics, e.g. Pluronic L121 (e.g. 1.25%), Triton-X, SDS, PEG, LTAB, Saponin, Cholate, etc.

Another optional component or additive, which may be contained in the inventive solution for transfection and/or injection as defined herein may be selected from the group of polymers or complexing agents, preferably to complex the nucleic acid (sequence) contained in the inventive solution for transfection and/or injection. Such polymers or complexing agents typically comprise, without being limited thereto, any polymer suitable for the preparation of a pharmaceutical composition, such as minor/major groove binders, nucleic acid binding proteins, lipoplexes, nanoplexes, non-cationic or non-polycationic compounds, such as PLGA, Polyacetate, Polyacrylate, PVA, Dextran, hydroxymethylcellulose, starch, MMP, PVP, heparin, pectin, hyaluronic acid, and derivatives thereof, or cationic or polycationic compounds, particularly cationic or polycationic polymers or cationic or polycationic lipids, preferably cationic or polycationic polymers. In the context of the present invention, such a cationic or polycationic compound is typically selected from any cationic or polycationic compound, suitable for complexing and thereby stabilizing a nucleic acid as defined herein, e.g. by associating the nucleic acid as defined herein with the cationic or polycationic compound. Particularly preferred, cationic or polycationic compounds are selected from cationic or polycationic peptides or proteins, including protamine, nucleoline, spermin or spermidine, or other cationic peptides or proteins, such as poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, plsl, FGF, Lactoferrin, Transportan, Buforin-2, Bac715-24, SynB, SynB(1), pVEC, hCT-derived peptides, SAP, protamine, spermine, spermidine, or histones. Additionally, preferred cationic or polycationic proteins or peptides may be selected from following proteins or peptides having the following total formula: (Arg)ₗ;(Lys)ₘ;(His)ₙ;(Orn)ₒ;(Xaa)ₓ, wherein I + m + n +o + x = 8-15, and I, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50% of all amino acids of the oligopeptide. Particularly preferred oligoarginines in this context are e.g. Arg₇, Arg₈, Arg₉, Arg₇, H₃R₉, R₉H₃, H₃R₉H₃, YSSR₉SSY, (RKH)₄, Y(RKH)₂R, etc. Further preferred cationic or polycationic compounds, which can be used for complexing the nucleic acid as defined herein may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cati-onic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers, such as PMOXA-PDMS copolymers, etc., blockpolymers consisting of a combination of one or more cationic blocks (e.g. selected of a cationic polymer as mentioned above) and of one or more hydrophilic- or hydrophobic blocks (e.g polyethyleneglycole); etc. Association or complexing the nucleic acid (sequence) of the inventive solution for transfection and/or injection as defined herein with cationic or polycationic compounds preferably provides adjuvant properties to the nucleic acid, preferably if provided as a RNA, and/or confers a stabilizing effect to the nucleic acid as defined herein by complexation and/or improves the transfection efficiency of the nucleic acid as defined herein. The procedure for stabilizing the nucleic acid as defined herein is in general described in EP-A-1083232, the disclosure of which is incorporated by reference into the present invention in its entirety. Particularly preferred as cationic or polycationic compounds are compounds selected from the group consisting of protamine, nucleoline, spermin, spermidine, oligoarginines as defined herein, such as Arg₇, Arg₈, Arg₉, Arg₇, H₃R₉, R₉H₃, H₃R₉H₃, YSSR₉SSY, (RKH)₄, Y(RKH)₂R, etc. Preferably, the nucleic acid of the inventive solution for transfection and/or injection as defined herein, preferably a RNA or mRNA, is complexed with a cationic or polycationic compound as defined herein.

As a further optional component, the inventive solution for transfection and/or injection as defined herein may additionally contain water, water for injection (WFI), or a buffer, preferably selected from a buffer as defined herein, e.g. a buffer containing 2-hydroxypropanoic acid, preferably including at least one of its optical isomers L-(+)-lactic acid, (*S*)-lactic acid, D-(-)-lactic acid or (*R*)-lactic acid, more preferably its biologically active optical isomer L-(+)-lactic acid, or a salt or an anion thereof, preferably selected from sodium-lactate, potassium-lactate, or Al₃⁺-lactate, NH₄⁺-lactate, Fe-lactate, Li-lactate, Mg-lactate, Ca-lactate, Mn-lactate or Ag-lactate, or a buffer selected from Ringer's lactate (RiLa), lactated Ringer's solution (main content sodium lactate, also termed "Hartmann's Solution" in the UK), acetated Ringer's solution, or ortho-lactate-containing solutions (e.g. for injection purposes), or lactate containing water. A buffer as defined herein may also be or resemble an isotonic buffer or solution, preferably selected from isotonic saline, a lactate or ortho-lactate-containing isotonic solution, a isotonic buffer or solution selected from phosphate-buffered saline (PBS), TRIS-buffered saline (TBS), Hank's balanced salt solution (HBSS), Earle's balanced salt solution (EBSS), standard saline citrate (SSC), HEPES-buffered saline (HBS), Grey's balanced salt solution (GBSS), or normal saline (NaCl), hypotonic (saline) solutions with addition of glucose or dextrose, or any solution as defined herein, etc. Isotonic buffers or solutions are particularly preferred as buffers in the context of the present invention for injection and/or transfection purposes. These isotonic buffers or solutions are preferably prepared by a skilled person preferably as defined herein or according to definitions preparation protocols well known in the art for these specific isotonic buffers or solutions. More preferably, the inventive solution for transfection and/or injection as defined herein may contain these (isotonic buffers) or solutions or (all) its contents in isotonic concentrations, preferably as defined herein or in the art for these specific isotonic solutions, e.g. to form an isotonic solution as described herein. In the above context a buffer may be used, more preferably an aqueous (isotonic) buffer or solution, containing a sodium salt, preferably at least 50 mM of a sodium salt, a calcium salt, preferably at least 0.01 mM of a calcium salt, and optionally a potassium salt, preferably at least 3 mM of a potassium salt. According to a preferred aspect, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCl, Kl, KBr, K₂CO₃, KHCO₃, K₂SO₄, and examples of calcium salts include e.g. CaCl₂, Cal₂, CaBr₂, CaCO₃, CaSO₄, Ca(OH)₂. Typically, the salts are present in such an (isotonic) buffer or solution, e.g. the inventive solution for transfection and/or injection as defined herein, in a concentration of at least 50 mM sodium chloride (NaCl), at least 3 mM potassium chloride (KCI) and at least 0.01 mM calcium chloride (CaCl₂). Furthermore, organic anions of the aforementioned cations may be contained in the (isotonic) buffer or solution. According to a more preferred aspect, the (isotonic) buffer or solution may contain salts selected from sodium chloride (NaCl), calcium chloride (CaCl₂) and optionally potassium chloride (KCl), wherein further anions may be present additional to the chlorides. CaCl₂ can also be replaced by another salt like KCl. The (isotonic) buffer or solution, e.g. the inventive solution for transfection and/or injection as defined herein, may be hypertonic, isotonic or hypotonic with reference to the specific reference medium, i.e. it may have a higher, identical or lower salt content with reference to the specific reference medium, wherein preferably such concentrations of the aforementioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Furthermore, according to a particularly preferred aspect, the nucleic acid (sequence) of the inventive solution for transfection and/or injection as defined herein, if lyophilized, may again be reconstituted after lyophilization in water or an (isotonic) buffer or solution as defined herein, e.g. to obtain the desired salt concentration or alternatively the desired buffer conditions.

As another optional component, the inventive solution for transfection and/or injection as defined herein may additionally contain an adjuvant. Such an adjuvant is preferably an immunostimulating agent, particularly a ligand of a pattern recognition receptor, or may be selected from cationic or polycationic compounds which are suitable for depot and delivery, or may be selected from immunostimulatory nucleic acids as defined herein.

A further optional component, the inventive solution for transfection and/or injection as defined herein may additionally contain a protein or a peptide, which may be selected, without being restricted thereto, e.g. from therapeutically active proteins or peptides, from antigens, e.g. tumor antigens, pathogenic antigens (e.g. selected from pathogenic proteins as defined herein or from animal antigens, viral antigens, protozoal antigens, bacterial antigens, allergenic antigens), autoimmune antigens, or further antigens, from allergens, from antibodies, from immunostimulatory proteins or peptides, from antigen-specific T-cell receptors, or from any other protein or peptide suitable for a specific (therapeutic) application. These proteins or peptides are preferably as defined above.

As another optional component, the inventive solution for transfection and/or injection as defined herein may additionally contain one or more compatible solid or liquid fillers or diluents or encapsulating compounds, which are suitable for administration to a patient to be treated. The term "compatible" as used here means that these constituents are capable of being mixed with the nucleic acid (sequence) of the inventive solution for transfection and/or injection as defined herein in such a manner that no interaction occurs which would substantially reduce the pharmaceutical effectiveness of the nucleic acid under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The inventive solution for transfection and/or injection as defined herein may occur as a liquid, a semi-liquid or even a semi-solid sample or solution, preferably as a liquid or a semi-liquid sample or solution, more preferably as a liquid sample or solution.

The pH of the inventive solution for transfection and/or injection as defined herein may be in the range of about 4 to 8, preferably in the range of about 6 to about 8, more preferably from about 7 to about 8.

Particularly preferred, the inventive solution for transfection and/or injection as defined herein is a transfection and/or injection solution. In this context, the inventive solution for transfection and/or injection surprisingly allows to significantly enhance the rate of transfection and thus the expression of an encoded protein as defined herein, preferably of a protein, which is encoded by a nucleic acid (sequence) as defined herein and forming part of the inventive solution for transfection and/or injection. Such a transfection and/or injection solution may contain any components as defined herein for the inventive solution for transfection and/or injection. Alternatively or additionally, the inventive solution for transfection and/or injection may be formed as a pharmaceutical composition or as a vaccine as defined in the following or may contain components thereof. Most preferably, the inventive solution for transfection and/or injection, either as a transfection and/or injection solution, as a pharmaceutical composition or as a vaccine, may comprise or even resemble an (isotonic) buffer or solution as defined herein and e.g. may (additionally) contain different salts (e.g. 0.5 mM to 50 mM potassium, 13 mM to 250 mM sodium, 0.2 mM to 10 mM calcium, and 0.2 mM to 10 mM magnesium). Different (isotonic) buffers or solutions as defined above may be utilized for this purpose, e.g. PBS, HBSS, Ringer's lactate. The inventive solution for transfection and/or injection may be administered as described in the following for a pharmaceutical composition or vaccine.

According to a further embodiment, the present invention furthermore provides a pharmaceutical composition, comprising the inventive solution for transfection and/or injection as defined herein containing at least one nucleic acid (sequence) and (free) histidine, preferably in a concentration of about 1 to about 25 µM, eventually further components as defined herein and optionally a pharmaceutically acceptable carrier and/or vehicle.

As a first ingredient, the inventive pharmaceutical composition comprises the inventive solution for transfection and/or injection as defined herein containing a nucleic acid (sequence) and (free) histidine as defined herein, preferably in a concentration of about 1 to about 25 µM.

As a second ingredient the inventive pharmaceutical composition may comprise another class of compounds, which may be added to the inventive pharmaceutical composition in this context. Such a second ingredient may be selected from at least one pharmaceutically active component. A pharmaceutically active component in this context is a compound that has a therapeutic effect against a particular indication, preferably cancer diseases, auto-immune disease, allergies, infectious diseases, or a further disease as defined herein. Such compounds include, without implying any limitation, preferably compounds including, without implying any limitation, peptides or proteins (e.g. as defined herein), nucleic acids, (therapeutically active) low molecular weight organic or inorganic compounds (molecular weight less than 5000, preferably less than 1000), sugars, antigens or antibodies (e.g. as defined herein), therapeutic agents already known in the prior art, antigenic cells, antigenic cellular fragments, cellular fractions; modified, attenuated or de-activated (e.g. chemically or by irridation) pathogens (virus, bacteria etc.), etc.

Furthermore, the inventive pharmaceutical composition may comprise a pharmaceutically acceptable carrier and/or vehicle. In the context of the present invention, a pharmaceutically acceptable carrier typically includes the liquid or non-liquid basis of the inventive pharmaceutical composition. If the inventive pharmaceutical composition is provided in liquid form, the carrier will typically be pyrogen-free water; isotonic saline or buffered (aqueous) solutions, e.g phosphate, citrate etc. buffered solutions. Particularly for injection of the inventive pharmaceutical composition, water or preferably a buffer, more preferably an isotonic buffer, may be used as defined for the inventive solution for transfection and/or injection. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person and may be as defined herein. Most preferred are isotonic solutions as defined herein in general may be present in an osmolality comparable to that of blood plasma, preferably in the range as defined herein.

However, one or more compatible solid or liquid fillers or diluents or encapsulating compounds may be used as well for the inventive pharmaceutical composition, which are suitable for administration to a patient to be treated. The term "compatible" as used here means that these constituents of the inventive pharmaceutical composition are capable of being mixed with the nucleic acid (sequence) as defined herein in such a manner that no interaction occurs which would substantially reduce the pharmaceutical effectiveness of the inventive pharmaceutical composition under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The inventive pharmaceutical composition may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques. Most preferred is intradermal and transdermal administration.

Preferably, the inventive pharmaceutical composition may be administered by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or via infusion techniques. Sterile injectable forms of the inventive pharmaceutical compositions may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation of the inventive pharmaceutical composition.

The inventive pharmaceutical composition as defined herein may also be administered orally in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient, i.e. the at least one nucleic acid as defined herein of the inventive solution for transfection and/or injection as defined herein is combined with emulsifying and suspending agents. If desired, certain sweetening, flavoring or coloring agents may also be added.

The inventive pharmaceutical composition may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the inventive pharmaceutical composition may be formulated in a suitable ointment, containing the components as defined herein suspended or dissolved in one or more carriers. Carriers for topical administration include, but are not limited to, mineral oil, liquid petrolatum, white petrolatum, propylene glycol, polyoxyethylene, polyoxypropylene compound, emulsifying wax and water. Alternatively, the inventive pharmaceutical composition can be formulated in a suitable lotion or cream. In the context of the present invention, suitable carriers include, but are not limited to, mineral oil, sorbitan monostearate, polysorbate 60, cetyl esters wax, cetearyl alcohol, 2-octyldodecanol, benzyl alcohol and water.

The inventive pharmaceutical composition typically comprises a "safe and effective amount" of the components of the inventive pharmaceutical composition as defined herein, particularly of the at least one nucleic acid (sequence). As used herein, a "safe and effective amount" means an amount of the at least one nucleic acid (sequence) that is sufficient to significantly induce a positive modification of a disease or disorder as defined herein. At the same time, however, a "safe and effective amount" is small enough to avoid serious side-effects, that is to say to permit a sensible relationship between advantage and risk. The determination of these limits typically lies within the scope of sensible medical judgment. A "safe and effective amount" of the components of the inventive pharmaceutical composition, particularly of the at least one nucleic acid (sequence) will furthermore vary in connection with the particular condition to be treated and also with the age and physical condition of the patient to be treated, the body weight, general health, sex, diet, time of administration, rate of excretion, drug combination, the activity of the specific nucleic acid (sequence) employed, the severity of the condition, the duration of the treatment, the nature of the accompanying therapy, of the particular pharmaceutically acceptable carrier used, and similar factors, within the knowledge and experience of the accompanying doctor. The inventive pharmaceutical composition may be used for human and also for veterinary medical purposes, preferably for human medical purposes, as a pharmaceutical composition in general or as a vaccine.

According to a specific embodiment, the inventive pharmaceutical composition may be provided as a vaccine. Such an inventive vaccine is typically composed like the inventive pharmaceutical composition, i.e. it contains at least comprising the inventive solution for transfection and/or injection as defined herein containing at least one nucleic acid (sequence) and (free) histidine as defined herein and optionally a pharmaceutically acceptable carrier and/or vehicle. Further components may be as defined herein for the inventive pharmaceutical composition. The inventive vaccine preferably supports at least an innate immune response of the immune system of a patient to be treated. Additionally, the inventive vaccine furthermore may also elicit an adaptive immune response, preferably, if the at least one nucleic acid (sequence) of the inventive vaccine encodes any of the above mentioned antigens or any of the above mentioned proteins or peptides, which may elicit an adaptive immune response.

The inventive vaccine may also comprise a pharmaceutically acceptable carrier, adjuvant, and/or vehicle as defined herein for the inventive pharmaceutical composition. In the specific context of the inventive vaccine, the choice of a pharmaceutically acceptable carrier is determined in principle by the manner in which the inventive vaccine is administered. The inventive vaccine can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, and sublingual injections. More preferably, vaccines herein may be administered by an intradermal, subcutaneous, or intramuscular route. Inventive vaccines are therefore preferably formulated in liquid (or sometimes in solid) form. The suitable amount of the inventive vaccine to be administered can be determined by routine experiments with animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive vaccine is to be administered orally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

The inventive vaccine can additionally contain one or more auxiliary substances in order to further increase its immunogenicity. A synergistic action of the at least one nucleic acid sequence of the inventive vaccine and of an auxiliary substance, which may be optionally contained in the inventive vaccine as described above, is preferably achieved thereby. Depending on the various types of auxiliary substances, various mechanisms can come into consideration in this respect. For example, compounds that permit the maturation of dendritic cells (DCs). In general, it is possible to use as auxiliary substance any agent that influences the immune system in the manner of a "danger signal" (LPS, GP96, etc.) or cytokines, such as GM-CFS, which allow an immune response produced by the immune-stimulating adjuvant according to the invention to be enhanced and/or influenced in a targeted manner or adjuvants.

Further additives which may be included in the inventive vaccine are emulsifiers, such as, for example, Tween^{®}, wetting agents, such as, for example, sodium lauryl sulfate; colouring agents; taste-imparting agents, pharmaceutical carriers; tablet-forming agents; stabilizers; antioxidants; preservatives.

The inventive vaccine can also additionally contain any further compound, which is known to be immune-stimulating due to its binding affinity (as ligands) to human pattern recognition receptors, particularly Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13. In this context immunostimulating nucleic acids as defined above for the nucleic acid contained in the herein defined inventive solution for transfection and/or injection are preferably added as a further compound to the inventive vaccine.

Finally, another class of compounds, which may be added to an inventive vaccine in this context, may be selected from at least one pharmaceutically active component as defined herein for the inventive pharmaceutical composition.

According to a further aspect, the present invention provides several applications and uses of the herein defined inventive solution for transfection and/or injection containing a nucleic acid (sequence) and (free) histidine, or of the inventive pharmaceutical composition or of the inventive vaccine all preferably as defined herein.

According to one specific embodiment, the present invention is directed to the use of the herein defined inventive solution for transfection and/or injection containing a nucleic acid (sequence) and (free) histidine for transfection and/or injection. The transfection and/or injection may occur *in vivo, in vitro* or *ex vivo.*

According to one other specific embodiment, the present invention is directed to the use of the herein defined inventive solution for transfection and/or injection containing a nucleic acid (sequence) and (free) histidine in amounts as defined above for the preparation of an injection solution. More preferably, such an injection solution may be used to (significantly) enhance the transfection efficiency of the nucleic acid and or the expression of a protein encoded by the nucleic acid (sequence), whereby the encoded protein is preferably a protein as defined herein. Accordingly, the present invention may also be directed to the use of the herein defined inventive solution for transfection and/or injection containing a nucleic acid (sequence) and (free) histidine in amounts as defined above for the preparation of a pharmaceutical composition to (significantly) enhance the transfection efficiency of the nucleic acid and or the expression of a protein encoded by the nucleic acid (sequence), whereby the encoded protein is preferably a protein as defined herein. Such an injection solution (or a corresponding pharmaceutical composition or vaccine) may contain any components as defined herein for the inventive solution for transfection and/or injection. Alternatively or additionally, the inventive injection solution may be formed as a pharmaceutical composition or vaccine as defined herein or may contain components thereof. Preferably, the inventive injection solution may be formulated and/or administered as described in the following for a pharmaceutical composition or vaccine.

According to one other specific embodiment, the present invention is directed to the (first medical) use of the herein defined inventive solution for transfection and/or injection containing at least one nucleic acid (sequence) and (free) histidine, preferably in an amount as defined above. Accordingly, the present invention provides a solution as defined herein for transfection and/or injection for use as a medicament. The medicament may be in the form of a pharmaceutical composition or in the form of a vaccine as a specific form of pharmaceutical compositions, both preferably as defined herein.

According to another embodiment, the present invention is directed to the (second medical) use of the herein defined inventive solution for transfection and/or injection containing at least one nucleic acid (sequence) and (free) histidine, preferably in an amount as defined above, (or the use of the inventive pharmaceutical composition or the inventive vaccine) for the treatment of diseases as defined herein, preferably to the use of the inventive solution for transfection and/or injection containing a nucleic acid (sequence) and (free) histidine, or more preferably the inventive pharmaceutical composition or the inventive vaccine (for the preparation of a medicament) for the prophylaxis, treatment and/or amelioration of various diseases as defined herein, preferably selected from cancer or tumor diseases, infectious diseases, preferably (viral, bacterial or protozoological) infectious diseases, autoimmune diseases, allergies or allergic diseases, monogenetic diseases, i.e. (hereditary) diseases, or genetic diseases in general, diseases which have a genetic inherited background and which are typically caused by a single gene defect and are inherited according to Mendel's laws, cardiovascular diseases, neuronal diseases, or any further disease mentioned herein. The present invention is also directed to a herein defined inventive solution for transfection and/or injection for use in the prophylaxis, treatment and/or amelioration of diseases as defined herein.

The present invention also allows treatment of diseases, which have not been inherited, or which may not be summarized under the above categories. Such diseases may include e.g. the treatment of patients, which are in need of a specific protein factor, e.g. a specific therapeutically active protein as mentioned above. This may e.g. include dialysis patients, e.g. patients which undergo a (regular) a kidney or renal dialysis, and which may be in need of specific therapeutically active proteins as defined herein, e.g. erythropoietin (EPO), etc.

According to a final embodiment, the present invention also provides kits, particularly kit of parts. Such kits may contain e.g. the inventive solution for transfection and/or injection, the inventive pharmaceutical composition or the inventive vaccine, all as defined herein. Kit of parts may likewise contain e.g. the inventive solution for transfection and/or injection, the inventive pharmaceutical composition or the inventive vaccine, all as defined herein, but, however, preferably divided into different parts of the kit. As an example, the inventive solution for transfection and/or injection, the inventive pharmaceutical composition or the inventive vaccine may be prepared as a kit of parts, e.g. by incorporating into one or more parts of the kit components of the inventive solution for transfection and/or injection, the inventive pharmaceutical composition or the inventive vaccine as described herein as a dry formulation, i.e. devoid of any liquid component, or as a liquid formulation, and in at least one further separate part of the kit the remaining components either in liquid or in dry form. Such a kit of parts may e.g. comprise in one part of the kit a lyophilized nucleic acid (sequence) as defined herein, optionally in a further part of the kit histidine in an amount as defined above either in dry or in liquid form (e.g. solubilized in water (e.g. water for injection, WFI), a histidine containing buffer, an isotonic salt solution and/or a combination thereof), and in at least one further part of the kit the remaining components of the inventive solution for transfection and/or injection, the inventive pharmaceutical composition or the inventive vaccine as described herein, e.g. water (e.g. water for injection, WFI), a histidine containing buffer, an isotonic salt solution and/or a combination thereof. Alternatively, such a kit of parts may e.g. comprise in one part of the kit a solubilized nucleic acid (sequence) as defined herein (e.g. in water (e.g. water for injection, WFI), a histidine containing buffer, an isotonic salt solution and/or a combination thereof), optionally in the same or a further part of the kit histidine in an amount as defined above in solubilized form (e.g. in water (e.g. water for injection, WFI), a histidine containing buffer, an isotonic salt solution and/or a combination thereof), and in at least one further part of the kit the remaining components of the inventive solution for transfection and/or injection, the inventive pharmaceutical composition or the inventive vaccine as described herein, e.g. water (e.g. water for injection, WFI), a histidine containing buffer, an isotonic salt solution and/or a combination thereof. In the above kit of parts, all components of the different parts are preferably present in the entire kit in such an amount and concentration as to resemble together the inventive solution for transfection and/or injection, the inventive pharmaceutical composition or the inventive vaccine as described herein. Further components may be incorporated in such kits of parts as described above for the inventive solution for transfection and/or injection or as described above for the inventive pharmaceutical composition or as described above for the inventive vaccine e.g. in the dry part(s) of the kit (e.g. in lyophilized for, if nucleic acids are added), in the liquid part(s) of the kit, preferably in solubilized form, or in at least one separate part of the kit as a dry form and/or in a lyophilized (liquid) form. Such kits, preferably kits of parts, may be applied, e.g., for any of the above mentioned applications or uses. The kit may optionally contain technical instructions with information on the administration and dosage of the kit or its components, e.g. the nucleic acid (sequence of the inventive solution for transfection and/or injection).

A specific kit of parts herein may comprise in one or more parts of the kit an inventive solution for transfection and/or injection as defined herein, optionally in one or more parts of the kit at least one therapeutically active protein, antibody or antigen as defined herein, further optionally in one ore more parts of the kit an adjuvant as defined herein, and also further optionally in one or more parts of the kit water (e.g. WFI), an isotonic buffer or solution or components thereof as defined herein, and optionally technical instructions with information on the administration and dosage of the kit or its components. The nucleic acid (sequence) may be lyophilized and the nucleic acid sequence and the histidine may be present in different parts of the kit.

Any of the herein defined aspects, modifications or (alternative) embodiments of the present invention may be combined with each other as suitable or necessary, provided, these combinations do not interfere with each other.

### Figures:

The following figures are intended to illustrate the invention further. They are not intended to limit the subject matter of the invention thereto.

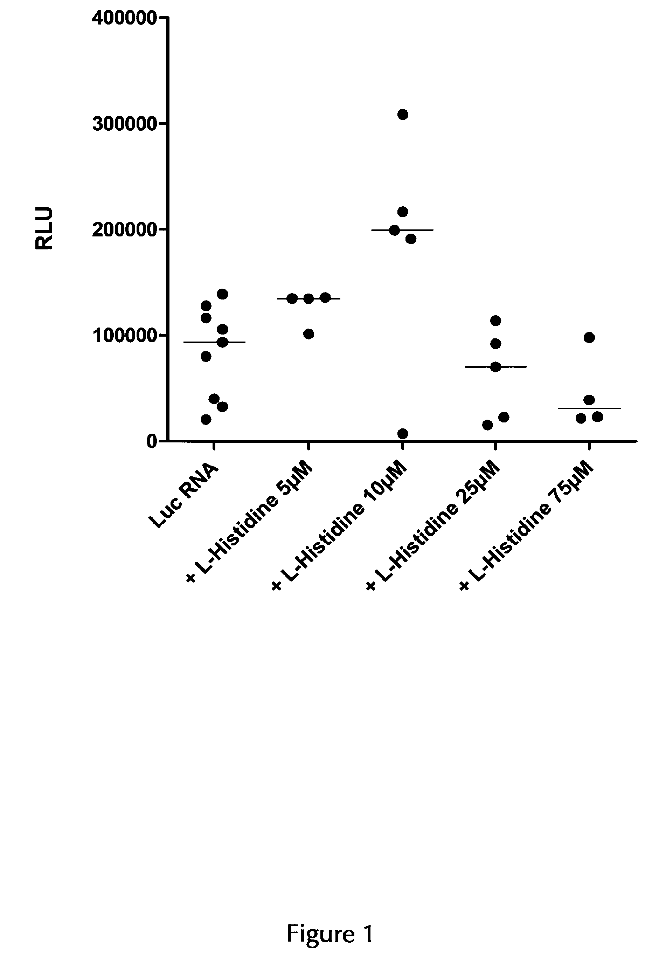

- Figure 1:: shows the *in vivo* luciferase expression in balb/c mice after intradermal injection of the following solutions 1) Luc RNA: 2,5 µg mRNA coding for luciferase in Ringer's lactate solution 2) + 5 µM Histidine: Luc RNA: 2,5 µg mRNA coding for luciferase in Ringer's lactate solution containing 5 µM histidine 3) + 10 µM Histidine: Luc RNA: 2,5 µg mRNA coding for luciferase in Ringer's lactate solution containing 10 µM histidine 4) + 25 µM Histidine: Luc RNA: 2,5 µg mRNA coding for luciferase in Ringer's lactate solution containing 25 µM histidine 5) + 75 µM Histidine: Luc RNA: 2,5 µg mRNA coding for luciferase in Ringer's lactate solution containing 75 µM histidine.

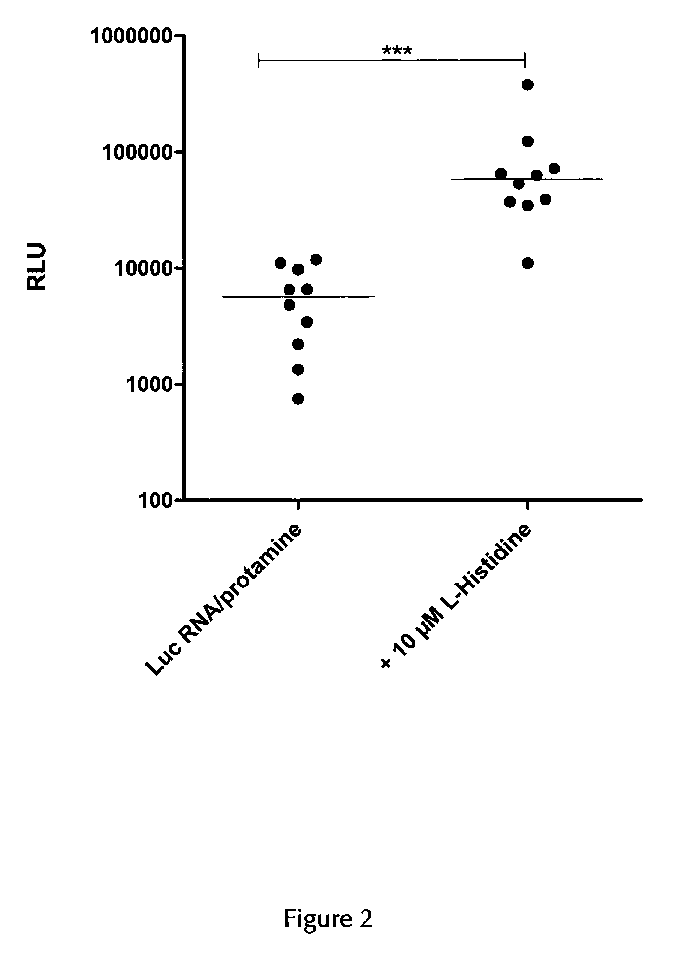

- Figure 2:: shows the *in vivo* luciferase expression in balb/c mice after intradermal injection of the following solutions 1) Luc RNA/protamine: 2,5 µg protamine complexed mRNA and 2,5 µg uncomplexed mRNA coding for luciferase in Ringer's lactate solution 2) + 10 µM Histidine: Luc RNA: 2,5 µg protamine complexed mRNA and 2,5 µg uncomplexed mRNA coding for luciferase in Ringer's lactate solution containing 10 µM histidine.

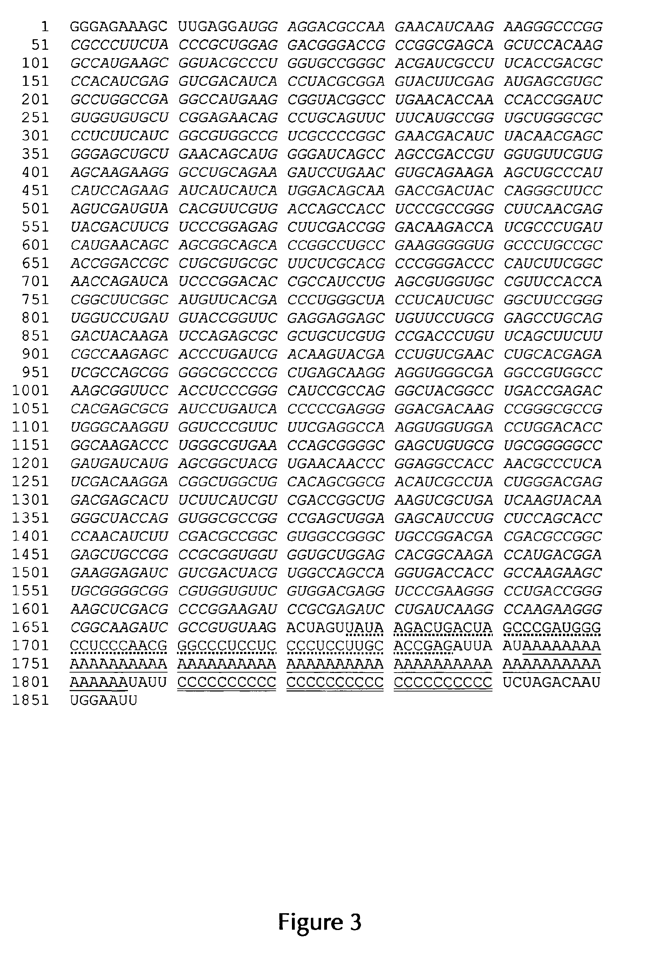

- Figure 3:: illustrates the mRNA sequence termed pCV19-Pp luc(GC)-muag-A70-C30 (SEQ ID NO: 1), coding for *Photinus pyralis* luciferase, which exhibits a length of 1857 nucleotides. The mRNA sequence contains following sequence elements:
• the coding sequence encoding *Photinus pyralis* luciferase;
• stabilizing sequences derived from alpha-globin-3'-UTR (muag (mutated alpha-globin-3'-UTR));
• 70 × adenosine at the 3'-terminal end (poly-A-tail);
• 30 × cytosine at the 3'- terminal end (poly-C-tail).
The ORF is indicated in italic letters, muag (mutated alpha-globin-3'-UTR) is indicated with a dotted line, the poly-A-tail is underlined with a single line and the poly-C-tail is underlined with a double line.

### Examples:

The following examples are intended to illustrate the invention further. They are not intended to limit the subject matter of the invention thereto.

### Example 1 - Preparation of plasmids

For the present examples DNA sequences encoding *Photinus pyralis* luciferase were prepared and used for subsequent *in vitro* transcription reactions and expression studies.

The DNA sequence corresponding to pCV19-Ppluc(GC)-muag-A70-C30 and encoding the *Photinus pyralis* luciferase coding sequence was prepared by modifying the wild type *Photinus pyralis* luciferase encoding DNA sequence by introducing a GC-optimized sequence for a better codon usage and stabilization. The stabilizing sequences were derived from alpha-globin-3'-UTR (muag (mutated alpha-globin-3'-UTR)). Furthermore, a stretch of 70 x adenosine was inserted at the 3'-terminal end (poly-A-tail) and a stretch of 30 x cytosine at the 3'- terminal end (poly-C-tail). The construct corresponded to SEQ ID NO: 1 (see Figure 3). The sequence of the final DNA construct had a length of 1857 nucleotides. The corresponding mRNA sequence was termed "pCV19-Ppluc(GC)-muag-A70-C30" (SEQ ID NO: 1) (see Figure 3).

The sequence contains following sequence elements:
- the coding sequence encoding *Photinus pyralis* luciferase (SEQ ID NO: 1);
- stabilizing sequences derived from alpha-globin-3'-UTR (muag (mutated alpha-globin-3'-UTR));
- 70 x adenosine at the 3'-terminal end (poly-A-tail);
- 30 x cytosine at the 3'- terminal end (poly-C-tail).

### Example 2 - In vitro transcription:

The respective DNA plasmids prepared according to Example 1 were transcribed *in vitro* using T7-Polymerase (T7-Opti mRNA Kit, CureVac, Tübingen, Germany) following the manufactures instructions. Subsequently the mRNA was purified using PureMessenger^{®} (CureVac, Tübingen, Germany).

### Example 4 - In vivo expression of the RNA constructs (see Figure 1)

### Constructs:

A first set of RNA containing (inventive) solutions for transfection and/or injection was prepared:
- 2,5 µg mRNA coding for luciferase in Ringer's lactate
- 2,5 µg mRNA coding for luciferase in Ringer's lactate containing 5 µM histidine
- 2,5 µg mRNA coding for luciferase in Ringer's lactate containing 10 µM histidine
- 2,5 µg mRNA coding for luciferase in Ringer's lactate containing 25 µM histidine
- 2,5 µg mRNA coding for luciferase in Ringer's lactate containing 75 µM histidine

### In vivo expression:

In a first set of experiments for determination of the *in vivo* expression of the RNA constructs each of the above prepared RNA containing (inventive) solutions for transfection and/or injection was administered into a separated group of mice. For these experiments, each group (4 to 10 mice per group) of 7 week old female balb/c mice were treated by intradermal injection with 100 µl of one the above prepared RNA containing (inventive) solutions for transfection and/or injection. After 24h mice were killed and the injected tissue was collected and lysed as described ahead. Tissue samples were crushed by a mill after freezing the samples in liquid nitrogen. Afterwards, the samples were lysed by adding 800 µl of lysing buffer (25 mM Tris HCL, 2 mM EDTA, 10%Glycerol, 1% Triton X-100, 2 mM DTT, 1 mM PMSF, pH 7.5-7.8). The lysates were shaken for 6 min and spun down for another 10 min at 4°C and 13500 rpm. The supernatants were measured with a luminometer LB9507 and analyzed by grouped analysis using 2-way ANOVA with Bonferroni post test. The results are shown in Figure 1.

### Results:

Figure 1 shows the *in vivo* luciferase expression in balb/c mice after intradermal injection of the following solutions 1) Luc RNA: 2,5 µg mRNA coding for luciferase in Ringer's lactate solution 2) solution 1) + 5 µM histidine: Luc RNA: 2,5 µg mRNA coding for luciferase in Ringer's lactate solution containing 5 µM histidine 3) solution 1) + 10 µM histidine: Luc RNA: 2,5 µg mRNA coding for luciferase in Ringer's lactate solution containing 10 µM histidine 4) solution 1) + 25 µM histidine: Luc RNA: 2,5 µg mRNA coding for luciferase in Ringer's lactate solution containing 25 µM histidine 5) solution 1) + 75 µM histidine: Luc RNA: 2,5 µg mRNA coding for luciferase in Ringer's lactate solution containing 75 µM histidine

### Discussion:

Expression of luciferase in balb/c mice was determined after intradermal injection of 2,5 µg mRNA coding for *Photinus pyralis* luciferase. The addition of (free) histidine to the injection solution increases the expression of the encoded protein up to 2 to 4 times compared to the control (1). Particularly the addition of histidine in a concentration of 5 µM (2) and 10 µM (3) increased the expression of the encoded protein.

### Example 5 - In vivo expression of the RNA constructs (see Figure 2)

### Constructs:

A second set of RNA containing (inventive) solutions for transfection and/or injection was prepared:
- mRNA coding for luciferase (2,5 µg mRNA complexed with protamine in a ratio of 2:1 (w/w) and 2,5 µg uncomplexed mRNA) partially complexed with protamine in Ringer's lactate
- mRNA coding for luciferase (2,5 µg mRNA complexed with protamine in a ratio of 2:1 (w/w) and 2,5 µg uncomplexed mRNA) partially complexed with protamine in Ringer's lactate containing 10 µM histidine

### In vivo expression:

In a second set of experiments for determination of *the in vivo* expression of the RNA constructs each of the above prepared RNA containing (inventive) solutions for transfection and/or injection was administered into a separated group of mice. For these experiments, each group (4 to 10 mice per group) of 7 week old female balb/c mice were treated by intradermal injection with 100 µl of one the above prepared RNA containing (inventive) solutions for transfection and/or injection. After 24h mice were killed and the injected tissue was collected and lysed as described ahead. Tissue samples were crushed by a mill after freezing the samples in liquid nitrogen. Afterwards, the samples were lysed by adding 800 µl of lysing buffer (25 mM Tris HCL, 2 mM EDTA, 10%Glycerol, 1% Triton X-100, 2 mM DTT, 1 mM PMSF, pH 7.5-7.8). The lysates were shaken for 6 min and spun down for another 10 min at 4°C and 13500 rpm. The supernatants were measured with a luminometer LB9507 and analyzed by grouped analysis using 2-way ANOVA with Bonferroni post test. The results are shown in Figure 2.

Figure 2 shows the *in vivo* luciferase expression in balb/c mice after intradermal injection of the following solutions 1) Luc RNA/protamine: 2,5 µg protamine complexed mRNA and 2,5 µg uncomplexed mRNA coding for luciferase in Ringer's lactate solution 2) + 10 µM Histidine: Luc RNA: 2,5 µg protamine complexed mRNA and 2,5 µg uncomplexed mRNA coding for luciferase in Ringer's lactate solution containing 10 µM histidine

### Discussion:

Expression of luciferase in balb/c mice was determined after intradermal injection of 2,5 µg mRNA complexed with protamine (2:1) (w/w) and 2,5 µg uncomplexed mRNA coding for luciferase. The addition of 10 µM (free) histidine (2) to the injection solution increases the expression of the encoded protein up to 10 times compared to the control (1).

## Claims

1. Solution for transfection and/or injection containing at least one nucleic acid sequence and (free) histidine in a concentration of about 1 to about 25 µM.

2. Solution according to claim 1, wherein the histidine concentration is in the range of about 5 to about 25 µM, or in the range of about 10 to about 20 µM, or in a concentration of about 10 µM.

3. Solution according to any of claims 1 or 2, wherein histidine is L-histidine.

4. Solution according to any of claims 1 to 3, wherein the solution is present in an osmolality in the range of 200 mosmol/I to about 400 mosmol/l.

5. Solution according to any of claims 1 to 4, wherein the solution additionally contains an isotonic buffer or its components wherein the isotonic buffer or its components are selected from Ringer's solution, Ringer's lactate solution, phosphate-buffered saline (PBS), TRIS-buffered saline (TBS), Hank's balanced salt solution (HBSS), Earle's balanced salt solution (EBSS), standard saline citrate (SSC), HEPES-buffered saline (HBS), Grey's balanced salt solution (GBSS), or normal saline (NaCl), or hypotonic (saline) solutions with addition of glucose or dextrose.

6. Solution according to claim 5 wherein the isotonic buffer or its components contains a sodium salt in a concentration of at least 50 mM of a sodium salt, a calcium salt in a concentration of at least 0.01 mM of a calcium salt, and optionally a potassium salt, in a concentration of at least 3 mM of a potassium salt, or sodium, calcium and, optionally, potassium salts in the form of their halogenides, including chlorides, iodides, or bromides, in the form of their hydroxides, carbonates, hydrogen carbonates, or sulfates, or include NaCl, Nal, NaBr, Na₂CO₃, NaHCO₃, Na₂SO₄, KCI, KI, KBr, K₂CO₃, KHCO₃, K₂SO₄, CaCl₂, Cal₂, CaBr₂, CaCO₃, CaSO₄, or Ca(OH)₂, or a buffer exhibiting salts selected from sodium chloride (NaCl), calcium chloride (CaCl₂) and optionally potassium chloride (KCI), present in a concentration of at least 50 mM sodium chloride (NaCl), at least 3 mM potassium chloride (KCI) and at least 0.01 mM calcium chloride (CaCl₂).

7. Solution according to any of claims 1 to 6, wherein the nucleic acid sequence is selected from DNA, including genomic DNA, single-stranded DNA molecules, double-stranded DNA molecules, coding DNA, DNA primers, DNA probes, immunostimulatory DNA, (short) DNA oligonucleotides ((short) oligodesoxyribonucleotides), or is selected from PNA (peptide nucleic acid) or is selected from RNA, including (short) RNA oligonucleotides ((short) oligoribonucleotides), a coding RNA, a messenger RNA (mRNA), an immunostimulatory RNA, a siRNA, an antisense RNA, a micro RNA or riboswitches, ribozymes aptamers, ribosomal RNA (rRNA), transfer RNA (tRNA), messenger RNA (mRNA), a viral RNA (vRNA), preferably RNA or mRNA.

8. Solution according to any of claims 1 to 7, wherein the nucleic acid sequence is a coding nucleic acid sequence, including a mRNA, encoding at least one therapeutically active protein or peptide, at least one tumor antigen, at least one pathogenic antigen or pathogen, at least one autoimmune (self-)antigen, at least one allergen or allergenic antigen, at least one antibody, at least one immunostimulatory protein or peptide or at least one antigen-specific T-cell receptor.

9. Solution according to any of claims 1 to 8 wherein the solution additionally contains complexing agents comprising lipoplexes, nanoplexes, cationic or polycationic compound, particularly cationic or polycationic polymers or cationic or polycationic lipids, including protamine, nucleoline, spermin or spermidine, or other cationic peptides or proteins, including poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia),* pAntp, plsl, FGF, Lactoferrin, Transportan, Buforin-2, Bac715-24, SynB, SynB(1), pVEC, hCT-derived peptides, SAP, protamine, spermine, spermidine, or histones, or proteins or peptides having the following total formula: (Arg)ₗ;(Lys)ₘ;(His)ₙ;(Orn)ₒ;(Xaa)ₓ, wherein I + m + n +o + x = 8-15, and I, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide, or are selected from cationic polysaccharides, including chitosan, polybrene, cationic polymers, polyethyleneimine (PEI), cationic lipids, DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, including β-aminoacid-polymers or reversed polyamides, modified polyethylenes, including PVP (poly(N-ethyl-4-vinylpyridinium bromide)), modified acrylates, including pDMAEMA (poly(dimethylaminoethyl methylacrylate)), modified Amidoamines including pAMAM (poly(amidoamine)), modified polybetaaminoester (PBAE), including diamine end modified 1,4 butanediol diacrylate-co-5-amino-1-pentanol polymers, dendrimers, including polypropylamine dendrimers or pAMAM based dendrimers, polyimine(s), including PEI: poly(ethyleneimine), poly(propyleneimine), polyallylamine, sugar backbone based polymers, including cyclodextrin based polymers, dextran based polymers, Chitosan, silan backbone based polymers, including PMOXA-PDMS copolymers, Blockpolymers consisting of a combination of one or more cationic blocks (e.g. selected of a cationic polymer as mentioned above) and of one or more hydrophilic- or hydrophobic blocks (e.g polyethyleneglycole)

10. Solution according to any of claims 1 to 9 wherein the solution additionally contains at least one therapeutically active protein, antibody and/or antigen.

11. Solution according to any of claims 1 to 10 wherein the solution additionally contains an adjuvant.

12. Solution according to any of claims 1 to 11 for use as a medicament.

13. Solution according to any of claims 1 to 11 for use in the prophylaxis, treatment and/or amelioration of diseases selected from cancer or tumor diseases, infectious diseases, including viral, bacterial or protozoological infectious diseases, autoimmune diseases, allergies or allergic diseases, monogenetic diseases, including hereditary diseases, or genetic diseases, diseases which have a genetic inherited background and which are typically caused by a single gene defect, cardiovascular diseases or neuronal diseases.

14. Pharmaceutical composition comprising the solution according to any of claims 1-11 and optionally a pharmaceutically acceptable carrier, and/or vehicle.

15. Pharmaceutical composition according to any of claim 14, wherein the pharmaceutical composition is a vaccine.

16. Use of a solution according to any of claims 1-11 for transfection and/or injection of the nucleic acid sequence, wherein the solution enhances the transfection efficiency of the nucleic acid and/or the expression of a protein encoded by the nucleic acid sequence.

17. A method for transfection of a cell or an organism, comprising the following steps:
a. preparing and/or providing a solution according to any of claims 1 to 11 or the pharmaceutical compositon according to claims 12 to 14;
b. transfecting a cell, a tissue or an organism using the solution or pharmaceutical composition prepared and/or provided according to step a..

18. Kit of parts, comprising the components of the solution as defined according to any of claims 1 to 11 in at least two different parts of the kit.

19. Kit of parts, according to claim 18 wherein the kit additionally comprises technical instructions with information on the administration and dosage of the kit or its components.

20. Kit of parts according to claim 18 to 19 wherein the nucleic acid sequence is lyophilized and the nucleic acid sequence and the histidine are present in different parts of the kit.
